# FASCICULE DE BREVET EUROPEEN

(11) **EP 1 706 090 B1**
(45) Date de publication et mention de la délivrance du brevet: **18.11.2009**
(21) Numéro de dépôt: 05717413.8
(22) Date de dépôt: 13.01.2005
(51) Int. Cl.: A61K 8/18, C09B 44/16

(54) **COLORANTS DIRECTS TRIAZOIQUES CATIONIQUES, COMPOSITION TINCTORIALE LES COMPRENANT ET PROCEDE DE COLORATION DE FIBRES KERATINIQUES LA METTANT EN OEUVRE**
DIREKTER TRIAZOISCHER KATIONISCHER FARBSTOFF, DIESE FARBSTOFFE ENTHALTENDE FÄRBENDE VERBINDUNG UND VERFAHREN ZUR FÄRBUNG VON KERATINFASERN MIT DIESEN FARBSTOFFEN
DIRECT TRIAZOIC CATIONIC DYESTUFF, TINCTING COMPOUND CONTAINING SAID DYESTUFFS AND METHOD FOR COLOURING KERATINIC FIBRES BY MEANS OF SAID DYESTUFFS

(30) Priorité: 13.01.2004 FR 0450073
(43) Date de publication de la demande: 04.10.2006
(73) Titulaire: L'ORÉAL, 75008 Paris (FR)
(72) Inventeur: GREAVES, Andrew, F-77144 Montevrain (FR)
(74) Mandataire: Wattremez, Catherine
(86) Numéro de dépôt international: PCT/FR2005/000078
(87) Numéro de publication internationale: WO 2005/073324

(56) Documents cités:
- EP-A- 0 714 954
- EP-A- 0 850 636
- WO-A-02/100834

## Description

La présente invention a pour objet des colorants directs triazoïques cationiques particuliers, une composition tinctoriale les comprenant, ainsi qu'un procédé de coloration de fibres kératiniques la mettant en oeuvre.

L'invention a trait au domaine de la coloration des fibres kératiniques humaines, et en particulier des cheveux. Plus particulièrement, elle concerne les colorations obtenues au moyen de compositions tinctoriales comprenant au moins un colorant direct.

Les colorants directs sont des substances colorées et colorantes qui ont une certaine affinité avec les fibres kératiniques humaines.

Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, parmi ceux dérivés du triarylméthane, ainsi que les colorants naturels, seuls ou en mélanges.

Parmi les colorants présentant l'avantage de conduire à des couleurs très chromatiques, on peut citer plus particulièrement les colorants de type azoïques comprenant au moins un hétérocycle, comme par exemple le colorant direct Basic Red 22 qui permet d'obtenir des couleurs rouge orangé.

L'inconvénient de ces colorants directs réside dans le fait que la résistance aux shampooings des couleurs obtenues est encore jugée trop faible.

L'un des buts de la présente invention est donc de proposer des colorants directs permettant d'accéder à des couleurs dont la résistance, notamment vis-à-vis des shampooings, est améliorée, sans diminuer les propriétés de montée du colorant dans la fibre ni de dégrader les caractéristiques de sélectivité du colorant.

Ainsi la présente invention a pour objet des colorants directs cationiques de formule (I) suivante : 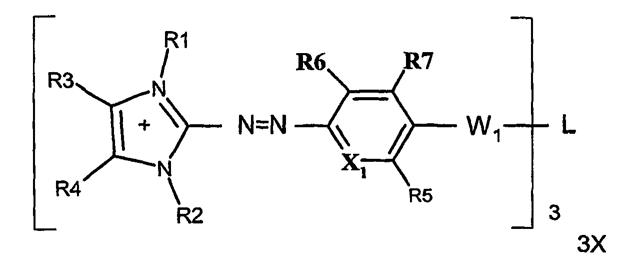 et leurs formes tautomères,
Formule dans laquelle
* W₁ représente -NR₈- ou -O- ;
* X₁ représente N ; CR₉ ;
   - R₁, R₂, identiques ou différents, représentent une chaîne hydrocarbonée ;
   - R₃, R₄ identiques ou différents, représentent un atome d'hydrogène, un atome d'halogène, un groupement nitro, un groupement cyano, une chaîne hydrocarbonée ;
   - R₅, R₆, R₇, R₈, R₉, identiques ou différents, représentant un atome d'hydrogène, une chaîne hydrocarbonée ;
   - L représente l'un des groupements ci-dessous : 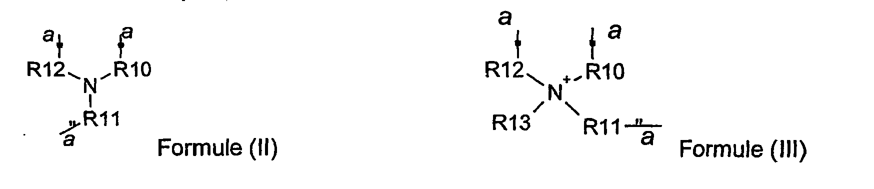 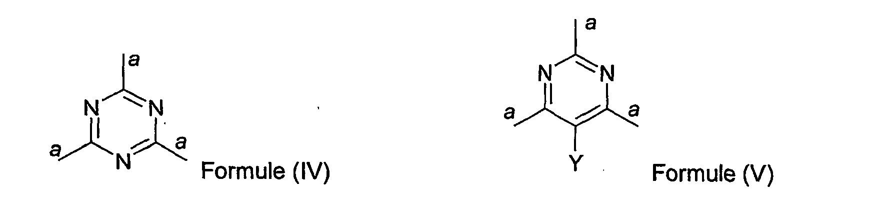 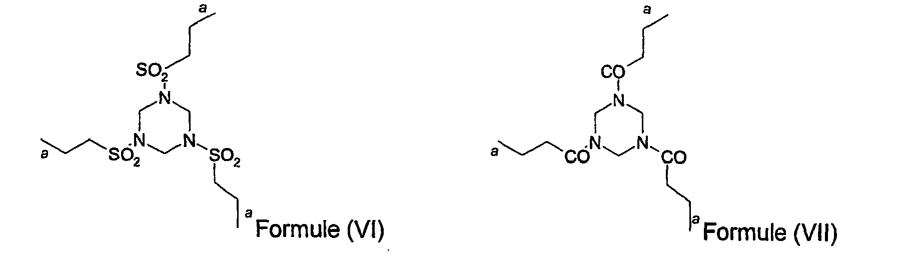
dans lesquelles :
- R₁₀, R₁₁, R₁₂, R₁₃, identiques ou différents, représentant une chaîne hydrocarbonée ;
- Y représente un atome d'halogène, de préférence le fluor ou le chlore ;
- la liaison a des formules (II) à (VII) est reliée au groupe W₁ de la formule (I) ;
   * X représente un anion organique ou minéral cosmétiquement acceptable.

Elle a de même pour objet une composition tinctoriale comprenant, dans un milieu approprié pour la coloration des fibres kératiniques humaines, au moins un colorant de formule (I) précitée.

Elle concerne de plus un procédé de coloration des fibres kératiniques humaines dans lequel on met en oeuvre les étapes suivantes :
a) on met en contact lesdites fibres, sèches ou humides, avec la composition tinctoriale selon l'invention, pendant une durée suffisante pour le développement de la coloration,
b) on rince éventuellement les fibres,
c) éventuellement on lave les fibres et on les rince,
d) on sèche les fibres ou on les laisse sécher.

L'invention a enfin pour objet les colorants de formule (I) précitée.

Mais d'autres avantages et caractéristiques de l'invention apparaîtront plus clairement à la lecture de la description et des exemples qui vont suivre.

Dans ce qui va suivre, lorsque des gammes sont décrites comme étant comprises entre deux valeurs, sauf indication contraire, ces valeurs sont incluses.

Comme indiqué auparavant, les colorants directs cationiques conformes à la présente invention sont présentés par les composés de formule (I).

Dans le texte, sauf indication contraire, lorsqu'il est fait référence à une chaîne hydrocarbonée, on désigne une chaîne hydrocarbonée :
- linéaire ou ramifiée, saturée ou insaturée en C₁-C₈, éventuellement interrompue par un ou plusieurs hétéroatomes tels que l'oxygène, l'azote ou le soufre, et/ou par un ou plusieurs groupements carbonyle ou SO₂, ladite chaîne ne comprenant pas un hétéroatome adjacent à un ou plusieurs hétéroatomes, ni un groupement carbonyle ou SO₂ adjacent à un ou plusieurs groupements carbonyle et/ou SO₂ ;
- et/ou aromatique en C₅- C₆;
- de plus la chaîne hydrocarbonée est éventuellement substituée par un ou plusieurs radicaux choisis parmi un radical hydroxyle ; un atome d'halogène, et de préférence le chlore, le fluor ; un radical alcoxy en C₁-C₄ ; un radical monohydroxy alcoxy dont la partie alkyle est en C₁-C₄, linéaire ou ramifiée, substituée ou non ; un radical polyhydroxy alcoxy, dans lequel la partie alkyle est en C₂-C₄, linéaire ou ramifiée, substituée ou non ; un radical amino substitué ou non par un ou plusieurs radicaux alkyle, identiques ou différents, en C₁-C₄, linéaires ou ramifiés, substitués ou non ; un radical thiol ; un radical alkylthio dont la partie alkyle est en C₁-C₄, linéaire ou ramifiée, substituée ou non ; un radical carboxylique ou forme acide ou salifiée (avec un métal alcalin ou un ammonium substitué ou non) ; un radical alcoxycarbonyle dont la partie alkyle est en C₁-C₄, linéaire ou ramifiée, substituée ou non ; un radical alkylamide dont la partie alkyle est en C₁-C₄, linéaire ou ramifiée, substituée ou non ; un radical alkylcarbamyle dont la partie alkyle est en C₁-C₄, linéaire ou ramifiée, substituée ou non ; un radical nitro ; un radical sulfonyle ; un radical alkylsulfonyle dont la partie alkyle est en C₁-C₄, linéaire ou ramifiée, substituée ou non ; un radical sulfonylamino, un radical alkylsulfonylamido dont la partie alkyle est en C₁-C₄, linéaire ou ramifiée, substituée ou non.

Il est à noter que les radicaux alkyle ou les parties alkyle des radicaux substituants peuvent eux-mêmes être substitués par un des radicaux listés ci-dessus.

Cependant, de préférence, les radicaux alkyle ou les parties alkyle de ces radicaux substituants ne sont pas substitués, ou bien sont substitués par un ou plusieurs radicaux choisis parmi les radicaux hydroxyle ; les atomes d'halogène comme par exemple le chlore, le fluor ; les radicaux alcoxy en C₁-C₄ ; les radicaux monohydroxy alcoxy dont la partie alkyle en C₁-C₄ ; les radicaux amino ; les radicaux amino substitués par un ou plusieurs radicaux alkyles, identiques ou non, en C₁-C₄.

Plus particulièrement, les radicaux R₁, R₂, identiques ou différents, représentent un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₈ ; un radical C₆-aryl (C₁-C₄)alkyle.

De manière particulièrement avantageuse, les radicaux R₁, R₂, identiques ou différents, représentent un radical alkyle en C₁-C₄, un radical benzyle.

Les radicaux R₁, R₂, identiques ou différents, représentent de préférence un radical méthyle, éthyle.

Selon un mode de réalisation particulier, les radicaux R₃, R₄, identiques ou différents, représentent un atome d'hydrogène ; un atome d'halogène, de préférence le chlore ; un groupement nitro ; un groupement cyano ; un radical alkyle en C₁-C₈ ou alcényle en C₂-C₈, éventuellement porteur d'un ou plusieurs radicaux hydroxyle, amino, amino substitué par un
ou plusieurs radicaux alkyles en C₁-C₈ éventuellement porteurs d'au moins un radical hydroxyle ; un radical alcoxy en C₁-C₈ ; un radical alkylthio en C₁-C₈ ; un radical sulfonylamino ; un radical phényle.

De manière plus avantageuse, lesdits radicaux R₃, R₄, identiques ou différents, représentent un atome d'hydrogène ; un atome de chlore ; un radical alkyle en C₁-C₈ ou alcényle en C₂-C₈, éventuellement porteur d'un ou plusieurs radicaux hydroxyle, amino, amino substitué par un ou plusieurs radicaux alkyle en C₁-C₈ éventuellement porteur d'au moins un radical hydroxyle, un radical alcoxy en C₁-C₈ ; un radical phényle.

De préférence, les radicaux R₃, R₄, représentent un atome d'hydrogène, un atome de chlore, un radical phényle.

En ce qui concerne les radicaux R₅, R₆, R₇, R₈, R₉, identiques ou différents, représentent plus particulièrement un atome d'hydrogène ; un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₈ ; un radical alkylthio en C₁-C₈ ; un radical sulfonylamino.

Avantageusement, les radicaux R₅, R₆, R₇, R₈, R₉, identiques ou différents, représentent un atome d'hydrogène, un radical alkyle en C₁-C₄ éventuellement porteur d'un
ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₄.

De préférence, les radicaux R₅, R₆, R₇, R₈, R₉, identiques ou différents, représentent un atome d'hydrogène, un radical alkyle en C₁-C₄.

Conformément à un mode de réalisation encore plus préféré de l'invention, les radicaux R₃, R₄, R₅, R₆, R₇, R₈, R₉, représentent un atome d'hydrogène.

Dans les formules (II) à (VII), les radicaux R₁₀, R₁₁, R₁₂, R₁₃, identiques ou différents, représentent plus particulièrement un atome d'hydrogène ; un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₈ ; un radical alkylthio en C₁-C₈, un radical sulfonylamino.

Lesdits radicaux R₁₀, R₁₁, R₁₂, R₁₃, selon un mode de réalisation préféré de l'invention, identiques ou différents, représentent un radical alkyle en C₁-C₄ éventuellement porteur d'un
ou plusieurs radicaux hydroxyle, amino, amino substitué par un ou plusieurs radicaux alkyles C₁-C₄ éventuellement porteur d'au moins un radical hydroxyle ; un radical alcoxy en C₁-C₄.

De manière encore plus préférée, les radicaux R₁₀, R₁₁, R₁₂, R₁₃, identiques ou différents, représentent un radical éthyle, n-propyle, n-butyle.

Conformément à un mode de réalisation particulier de l'invention, X₁ représente un groupement divalent CR₉, R₉ étant défini précédemment.

De préférence R₉ représente un atome d'hydrogène.

Par ailleurs, une variante particulièrement avantageuse de l'invention correspond à un colorant de formule (I) dans lequel W₁ représente un groupement divalent NR₈, R₈ ayant la même définition que celles données plus haut.

Plus particulièrement R₈ représente un radical alkyle en C₁-C₄ et de préférence un atome d'hydrogène,

Le colorant direct de formule (I) comprend en outre un anion cosmétiquement acceptable, de nature organique ou minérale.

A titre d'exemple d'anions de nature minérale, on peut citer notamment les halogénures, comme les chlorures ; les hydroxydes ; les sulfates ; les hydrogénosulfates.

A titre d'exemples d'anions de nature organique, conviennent les anions tels que l'acétate ; le citrate ; le tartrate ; les alkylsulfates pour lesquels la partie alkyle, linéaire ou ramifiée, est en C₁-C₆, comme l'ion méthosulfate, éthosulfate ; les alkylsulfonates pour lesquels la partie alkyle, linéaire ou ramifiée, est en C₁-C₆ ; les arylsulfonates pour lesquels la partie aryle, de préférence phényle est éventuellement substituée par un ou plusieurs radicaux alkyle en C₁-C₄.

Un mode de réalisation préféré de l'invention est constitué par des colorants directs de formule (I) choisis parmi les composés suivants :
- Trichlorure de 2-((E)-{4-[(3-{bis[3-({4-[(E)-(1,3-dimethyl-1H-imidazol-3-ium-2-yl) diazényl]phényl}amino)propyl]amino}propyl)amino]phényl}diazényl)-1,3-diméthyl-1H-imidazol-3-ium
- Tétrachlorure de 2-{(E)-[4-({3-[bis[3-({4-[(E)-(1,3-diméthyl-1H-imidazol-3-ium-2-yl) diazényl]phényl}amino)propyl](méthyl)ammonio]propyl}amino)phényl]diazényl}-1,3-diméthyl-1H-imidazol-3-ium
- Tétrachlorure de 2-((E)-{4-[{3-[bis{3-[{4-[(E)-(1,3-diméthyl-1H-imidazol-3-ium-2-yl)diazényl]phényl}(méthyl)amino]propyl}(méthyl)ammonio]propyl}(méthyl)amino]phé nyl}diazényl)-1,3-diméthyl-1H-imidazol-3-ium
- Tétrachlorure de 2-((E)-{4-[{3-[bis{3-[{4-[(E)-(1,3-diéthyl-1H-imidazol-3-ium-2-yl)diazényl]phényl}(éthyl)amino]propyl}(éthyl)ammonio]propyl}(ethyl)amino]phényl}di azényl)-1,3-diéthyl-1H-imidazol-3-ium
- Tétrachlorure de 2-((E)-{4-[{3-[bis{3-[{4-[(E)-(1,3-diméthyl-1H-imidazol-3-ium-2-yl)diazényl]phényl}(éthyl)amino]propyl}(éthyl)ammonio]propyl}(éthyl)amino]phényl}di azényl)-1,3-diméthyl-1H-imidazol-3-ium
- Trichlorure de 2-((E)-{4-[(3-{3,5-bis[3-({4-[(E)-(1,3-diméthyl-1H-imidazol-3-ium-2-yl)diazényl]phényl}amino)propanoyl]-1,3,5-triazinan-1-yl}-3-oxopropyl)amino]phényl} diazényl)-1,3-diméthyl-1H-imidazol-3-ium
- Trichlorure de 2-((E)-{4-[[3-(3,5-bis{3-[{4-[(E)-(1,3-dimtéhyl-1H-imidazol-3-ium-2-yl)diazényl]phényl}(éthyl)amino]propanoyl)-1,3,5-triazinan-1-yl)-3-oxopropyl](éthyl) amino]phényl}diazényl)-1,3-diméthyl-1H-imidazol-3-ium
- Trichlorure de 2-{(E)-[4-({2-[(3,5-bis{[2-({4-[(E)-(1,3-diméthyl-1H-imidazol-3-ium-2-yl)diazényl]phényl}amino)éthyl]sulfonyl}-1,3,5-triazinan-1-yl) sulfonyl]éthyl}amino) phényl] diazényl}-1,3-diméthyl-1H-imidazol-3-ium
- Trichlorure de 2-((E)-{4-[(2-{[3,5-bis({2-[{4-[(E)-(1,3-diméthyl-1H-imidazol-3-ium-2-yl) diazényl]phényl}(éthyl)amino]éthyl}sulfonyl)-1,3,5-triazinan-1-yl]sulfonyl}éthyl)(éthyl) amino]phényl}diazényl)-1,3-dimethyl-1H-imidazol-3-ium
- Trichlorure de 2-[(E)-(4-{[4,6-bis({4-[(E)-(1,3-diméthyl-1H-imidazol-3-ium-2-yl) diazényl]phényl}amino)-1,3,5-triazin-2-yl]amino}phényl)diazényl]-1,3-diméthyl-1H-imidazol-3-ium
- Trichlorure de 2-((E)-{4-[{4,6-bis[{4-[(E)-(1,3-diméthyl-1H-imidazol-3-ium-2-yl) diazényl]phényl}(méthyl)amino]-1,3,5-triazin-2-yl}(méthyl)amino]phényl}diazényl)-1,3-diméthyl-1H-imidazol-3-ium
- Trichlorure de 2-[(E)-(4-{[5-chloro-2,6-bis({4-[(E)-(1,3-diméthyl-1H-imidazol-3-ium-2-yl)diazényl]phényl}amino)pyrimidin-4-yl]amino}phényl)diazényl]-1,3-diméthyl-1H-imidazol-3-ium
- Trichlorure de 2-((E)-{4-[{5-chloro-2,6-bis[{4-[(E)-(1,3-diméthyl-1H-imidazol-3-ium-2-yl)diazényl]phényl}(méthyl)amino]pyrimidin-4-yl}(méthyl)amino]phényl}diazényl)-1,3-diméthyl-1H-imidazol-3-ium.

Bien entendu, dans la liste ci-dessus, le contre-ion peut être changé et choisi parmi les anions cosmétiquement acceptables, de nature organique ou minérale, listés auparavant.

Ces colorants peuvent être synthétisés de manière classique, et l'on pourra se référer à l'ouvrage Advanced Organic Chemistry, March, 4éme Ed.

Cependant, à titre d'exemple, on peut mettre en oeuvre l'une des deux voies mentionnées ci-dessous : 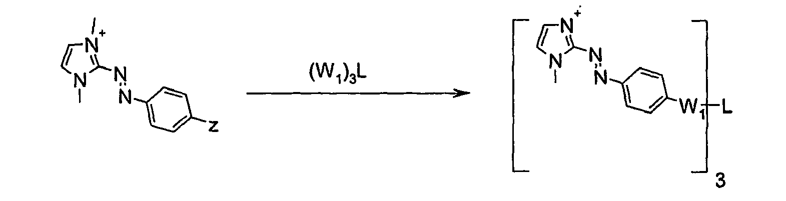 ou

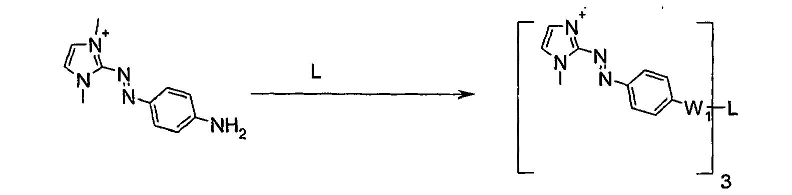

Dans ces formules, Z peut représenter un groupement de type -OR dans lequel R est un radical alkyle, linéaire ou ramifié, en C1-C4 ; -OS02R' avec R' représente un radical alkyle, linéaire ou ramifié, en C1-C4, un radical aryle en C6 ; un atome d'halogène.

La réaction de substitution peut être effectuée de manière tout à fait classique et l'on pourra se référer à l'ouvrage Advanced Organic Chemistry, March, 4éme Ed, pour avoir plus de détails sur les conditions opératoires mises en oeuvre.

Pour obtenir des composés dans lesquels L représente un radical de formule (VI) ou (VII), on peut, par exemple, mettre en oeuvre des réactions de Michael.

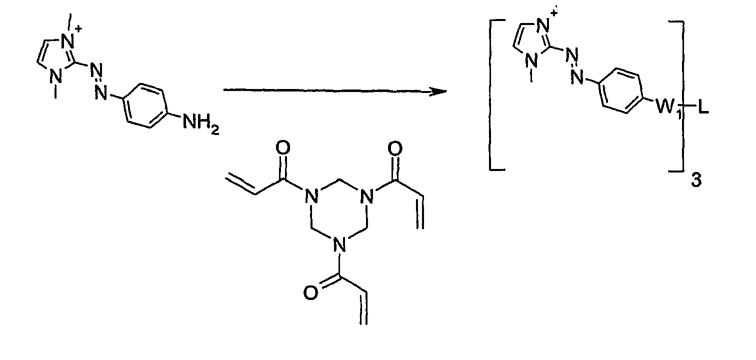

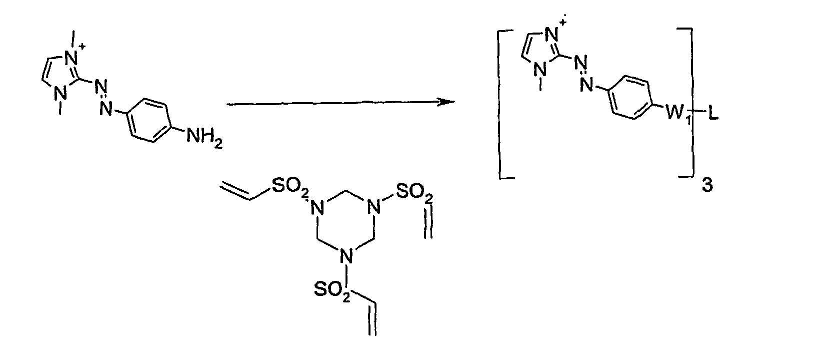

On peut mettre en oeuvre des réactions conventionnelles de substitution pour obtenir des composés dans lesquels L représente un radical de formule (IV) ou (V).

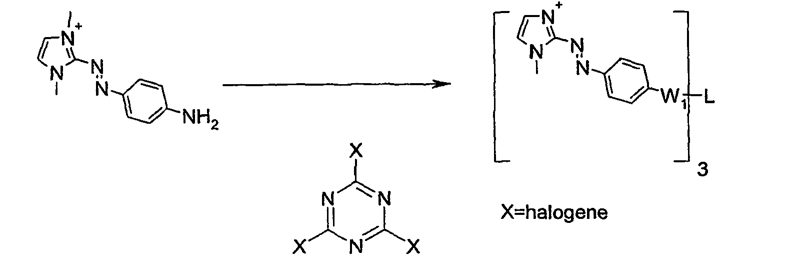

La présente invention a pour objet une composition tinctoriale comprenant, dans un milieu approprié pour la teinture des fibres kératiniques humaines, notamment les cheveux, au moins un colorant direct cationique de formule (I) qui vient d'être détaillé.

Plus particulièrement, la teneur en colorant de formule (I) est comprise entre 0,001 et 20% en poids par rapport au poids de la composition tinctoriale, de préférence entre 0,01 et 10% en poids par rapport au poids de la composition tinctoriale.

Il est à noter que la composition peut comprendre au moins un colorant direct additionnel différent de celui qui vient d'être décrit, au moins une base d'oxydation et/ou au moins un coupleur, ou leurs mélanges.

En ce qui concerne les colorants directs additionnels, on peut utiliser des espèces cationiques, anioniques ou non ioniques.

Avantageusement, ils peuvent être choisis parmi les colorants benzéniques nitrés, les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines (comme notamment les azacarbocyanine, diazacarbocyanine, diazahémicyanine, hémicyanine, tétraazacarbocyanine), diazines, dicétopyrrolopyrroles, dioxazines, diphénylamines, diphénylméthanes, dithiazines, flavanthrones, flavones, fluorindines, formazans, hydrazones, hydroxycétones, indamines, indanthrones, indigoides, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, styryles, tétrazoliums, thiazines, thioindigo, thiopyronines, triarylméthanes, xanthènes.

S'ils sont présents, la teneur en colorant(s) direct(s) dans la composition varie en général de 0,001 à 20% en poids par rapport à la composition tinctoriale, et de préférence de 0,01 à 10% du poids total de la composition tinctoriale.

En ce qui concerne les bases d'oxydation, elles peuvent être choisies notamment parmi les o-phénylènediamines, les p-phénylènediamines, les bis-phénylalkylènediamines, les o-aminophénols, les p-aminophénols, les bis-p-aminophénols, les bases hétérocycliques, leurs sels d'addition avec un acide ou une base, ainsi que leurs mélanges.

En général, lorsqu'elles sont présentes dans la composition, la teneur en base(s) d'oxydation représente de 0,0005 à 12% en poids par rapport au poids de la composition tinctoriale, avantageusement de 0,005 à 8% en poids par rapport au poids de la composition tinctoriale.

Quant aux coupleurs éventuellement associés aux bases d'oxydation précitées, on peut mettre en oeuvre un ou plusieurs composés choisis parmi les m-aminophénols, les m-phénylènediamines, les m-diphénols, les naphtols, les coupleurs hétérocycliques, leurs sels d'addition avec un acide, ainsi que leurs mélanges.

Lorsqu'ils sont présents, ces coupleurs représentent plus spécialement de 0,0001 à 10% en poids par rapport au poids de la composition tinctoriale, et de préférence de 0,005 à 5% en poids par rapport au poids de la composition tinctoriale.

D'une manière générale, les sels d'addition avec un acide ou avec une base, des bases d'oxydation et coupleurs sont notamment choisis les sels d'addition avec l'acide chlorhyddrique, bromhydrique, sulfurique, tartrique, lactique, acétique, et les sels d'addition avec l'hydroxyde sodium, de potassium, l'ammoniaque, les amines, les alcanolamines.

La composition selon l'invention peut de plus comprendre au moins un tensioactif, de préférence non ionique, anionique, amphotère ou zwittérionique.

Parmi les tensioactifs non ioniques, on peut citer les alcools, les alpha-diols, les alkylphénols polyéthoxylés et/ou polypropoxylés, ayant une chaîne hydrocarbonée comprenant par exemple de 8 à 30 atomes de carbone, de préférence de 8 à 18 atomes de carbone, le nombre de groupements oxyde d'éthylène et/ou oxyde de propylène pouvant aller notamment de 2 à 50.

On peut également citer les copolymères d'oxyde d'éthylène et de propylène, les condensats d'oxyde d'éthylène et/ou de propylène sur des alcools gras ; les amides gras polyéthoxylés ayant de préférence de 2 à 30 moles d'oxyde d'éthylène ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, etc.

Parmi les tensioactifs anioniques, on peut citer entre autres les sels (en particulier sels alcalins, notamment de sodium, de magnésium, les sels d'ammonium, sels d'amines ou d'aminoalcools) d'alkylsulfates, alkyléthersulfates, alkylamidoéthersulfates, alkylarylpolyéther sulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄) sulfosuccinates, alkyl(C₆-C₂₄) éthersulfosuccinates, alkyl(C₆-C₂₄) amidesulfosuccinates ; alkyl(C₆-C₂₄) sulfoacétates ; acyl(C₆-C₂₄) sarcosinates et glutamates ; esters d'alkyl(C₆-C₂₄)polyglycosides carboxyliques ; alkylsulfosuccinamates ; acyliséthionates et N-acyltaurates ; le radical alkyle ou acyle de tous ces différents composés comportant de préférence de 12 à 20 atomes de carbone, et le radical aryle désignant de préférence un groupement phényle ou benzyle.

Conviennent aussi les sels d'acides gras tels que les sels des acides oléique, ricinoléique, palmitique, stéarique, les acides d'huile de coprah hydrogénée ou non ; les acides d'alkyl D-galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène notamment d'éthylène, et leurs mélanges.

En ce qui concerne les tensioactifs amphotères ou zwittérioniques conviennent notamment les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer amphocarboxyglycinates et amphocarboxypropionates, comme par exemple les disodium cocoamphodiacetate, disodium lauroamphodiacetate, disodium caprylamphodiacetate, disodium cocoamphodipropionate, disodium lauroamphodipropionate, etc. (produits de la gamme Miranol®, notamment Miranol^{®} C2M concentré, commercialisés la société Rhodia Chimie).

La teneur en tensioactif dans la composition représente habituellement de 0,01 à 40 % en poids et de préférence de 0,5 à 30 % en poids, du poids total de la composition.

La composition tinctoriale conforme à l'invention peut également comprendre divers adjuvants utilisés classiquement, tels que des polymères anioniques, cationiques, non ioniques, amphotères, zwittérioniques, des agents épaississants minéraux, des agents antioxydants, des agents de pénétration, des parfums, des tampons, des agents dispersants, des agents de conditionnement tels que par exemple des cations, des silicones volatiles ou non volatiles, modifiées ou non modifiées, des polymères amphiphiles cationiques, des agents filmogènes, des céramides, des vitamines ou provitamines, des agents conservateurs, des agents stabilisants, des agents opacifiants ou matifiants comme le dioxyde de titane, des charges minérales, comme les argiles, les silices notamment pyrogénées à caractère hydrophile ou hydrophobe, des filtres solaires, etc.

Les adjuvants cités ci-dessus sont en général présents en quantité comprise, pour chacun d'eux, entre 0,01 et 20% en poids par rapport au poids de la composition.

Le milieu de la composition tinctoriale est un milieu cosmétiquement acceptable.

Il est de préférence constitué par un milieu aqueux comprenant de l'eau et éventuellement un ou plusieurs solvants organiques acceptables sur le plan cosmétique. Plus particulièrement, les solvants sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl - 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C1-C4, comme par exemple, le monoéthyléther ou le monobutyléther du diéthylèneglycol, seuls ou en mélange.

Le ou les solvants usuels décrits ci-dessus, s'ils sont présents, représentent habituellement de 1 à 40 % en poids, plus préférentiellement de 5 à 30 % en poids, par rapport au poids total de la composition tinctoriale.

La composition selon l'invention peut en outre comprendre au moins un agent oxydant.

Habituellement, l'agent oxydant est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins ; les enzymes telles que, par exemple, les peroxydases et les oxydo-réductases à deux ou à quatre électrons, seuls ou en mélanges.

La teneur en agent oxydant, s'il est présent dans la composition tinctoriale selon l'invention, est en général comprise entre 1 et 20 % en poids par rapport au poids de la composition tinctoriale, de préférence entre 1 et 12 % en poids par rapport au poids de la composition tinctoriale.

Il est à noter que cette variante est appropriée lorsque l'on souhaite observer, en plus de l'effet de coloration obtenu entre autre par le colorant direct selon l'invention, un effet d'éclaircissement des fibres.

Cette même variante est de plus mise en oeuvre lorsque la composition tinctoriale selon l'invention comprend au moins une base d'oxydation et éventuellement un coupleur.

Le pH de la composition tinctoriale selon l'invention est généralement compris entre 3 et 12 environ, et de préférence entre 5 et 11 environ.

Il peut être ajusté à la valeur désirée au moyen d'agents acidifiants ou alcalinisants.

Parmi les agents acidifiants, on peut citer, à titre d'exemples, les acides minéraux ou organiques comme l'acide chlorhydrique, l'acide orthophosphorique, l'acide sulfurique, l'acide acétique.

Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 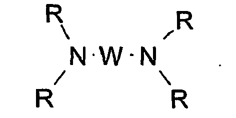 dans laquelle W est un reste propylène éventuellement substitué par un groupement hydroxyle ou un radical alkyle en C₁-C₆ les radicaux R, identiques ou différents, représentent un atome d'hydrogène, un radical alkyle en C₁-C₆ ou hydroxyalkyle en C₁-C₆.

Notons que la composition peut se trouver sous la forme d'une lotion, épaissie ou non, d'une crème, d'un gel, d'une mousse, d'un spray ou de toute autre forme appropriée pour l'application ultérieure de cette composition.

Un procédé de coloration des fibres kératiniques humaines, en particulier telles que les cheveux, mettant en oeuvre la composition selon l'invention, va maintenant être décrit.

Ainsi, on peut mettre en oeuvre les étapes suivantes :
a) on met en contact lesdites fibres, sèches ou humides, avec la composition tinctoriale selon l'invention, pendant une durée suffisante pour le développement de la coloration,
b) on rince éventuellement les fibres,
c) éventuellement on lave les fibres et on les rince,
d) on sèche les fibres ou on les laisse sécher.
   Dans le cas où la composition tinctoriale comprend au moins un agent oxydant, la composition appliquée sur les fibres lors de l'étape a) est de préférence obtenue par mélange extemporané de la composition selon l'invention sans l'agent oxydant, avec une composition comprenant au moins ledit agent oxydant.

De préférence, cet agent oxydant est le peroxyde d'hydrogène.

De manière avantageuse, la composition oxydante est du type de celles utilisées classiquement dans le domaine de la coloration.

La composition ainsi obtenue est donc appliquée sur les fibres kératiniques, sèches ou humides, puis laissée pendant un temps de pose suffisant pour obtenir la coloration recherchée.

Le temps de pose est en général d'environ 5 à 60 minutes et plus particulièrement d'environ 5 à 40 minutes.

La température nécessaire au développement de la coloration est généralement comprise entre la température ambiante (15 à 25°C) et 80°C et plus particulièrement entre 15 et 40°C.

Une fois cette étape réalisée, on rince éventuellement les fibres, éventuellement on les lave avec un shampooing et on les rince. Puis on sèche les fibres ou on les laisse sécher.

La présente invention a enfin pour objet un dispositif à plusieurs compartiments dans lequel un premier compartiment contient une composition tinctoriale comprenant au moins un colorant triazoïque cationique de formule (I) décrit auparavant, et un deuxième compartiment contient un agent oxydant.

Des exemples concrets mais non limitatifs de l'invention vont maintenant être présentés.

### EXEMPLES 1 - SYNTHESES

### 1/ Synthèse d'un composé de formule (I) suivante :

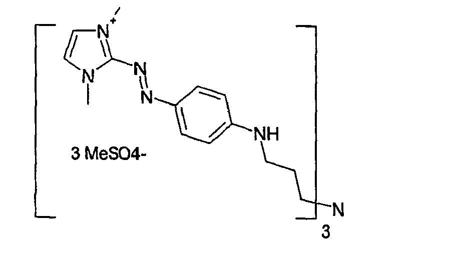

### Schéma réactionnel général :

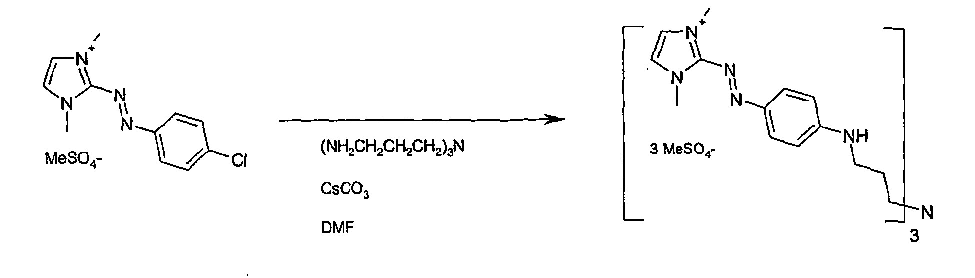

### Synthèse :

On dissout 4,68 g de méthosulfate de 2-[(4-chlorophényl)diazényl]1,3-diméthyl-1H-imidazol-3-ium dans 50 ml de diméthylformamide.

On ajoute ensuite 5 g de carbonate de césium, puis 0,5 g de tris(3-aminopropyl)amine.

Le mélange obtenu est conservé sous agitation à 80°C pendant 2 heures puis on ajoute à nouveau 0,5 g de tris(3-aminopropyl)amine.

On ajoute 0,5 g de tris(3-aminopropyl)amine après 16 heures sous agitation à 80°C, et on laisse dans ces conditions de température et sous agitation pendant 4 heures.

La solution résultante est ensuite refroidie à température ambiante, filtrée puis on évapore le solvant pour obtenir un composé huileux de couleur rouge.

Le produit est ensuite purifié sur une colonne HPLC.

Les analyses confirment la structure ci-dessus du composé obtenu.

### 2/ Synthèse du composé comparatif de formule suivante :

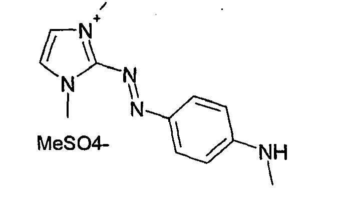

### Schéma réactionnel général :

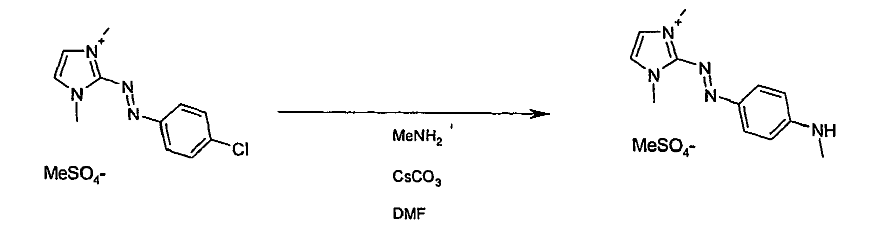

### Synthèse :

On dissout 1 g de méthosulfate de 2-[(4-chlorophényl)diazényl]1,3-diméthyl-1H-imidazol-3-ium dans 50 ml de 2-propanol, puis on ajoute 2 g de méthylamine.

Le mélange est maintenu sous agitation au reflux pendant 4 heures puis refroidi à température ambiante, filtré.

On évapore le solvant afin d'obtenir un solide rouge.

Le produit est ensuite purifié sur une colonne (chromatographie liquide ; silice ; éluant : méthanol / dichlorométhane).

Les analyses confirment la structure ci-dessus du composé obtenu.

### EXEMPLES 2 - APPLICATION

### 1/ Coloration

On prépare une solution tamponnée à pH 9 par mélange de 2 g d'acétate d'ammonium dans 40 ml d'eau. Le pH est ajusté par addition d'ammoniaque et le volume est complété à 100 ml par addition d'eau désionisée.

On dissout 5.10⁻⁴ mol % de chacun des colorants obtenus lors de l'exemple 1 précédent, dans la solution tamponnée précitée.

On met en contact une mèche de cheveux blancs avec la solution résultante avec un rapport de bain de 10 pour 1.

Après 20 minutes de pose, la mèche est rincée à l'eau désionisée pour éliminer l'excès de solution de colorant.

On obtient pour chacun des deux colorants, une mèche de cheveu rouge.

### 2/ Test de shampoing

Chaque mèche de cheveux colorée selon l'étape précédente est lavée à la main avec une solution comprenant 1 % en volume de shampooing, pendant 30 secondes, puis rincée avec 200 ml d'eau.

Le procédé est répété 6 fois.

### 3/ Résultats

Les mèches obtenues dans les deux cas ont conservé la même couleur mais l'intensité de la couleur de la mèche colorée avec le composé conforme à l'invention est visuellement plus importante que celle de la mèche colorée avec un composé n'entrant pas dans le cadre de l'invention. Cela montre par conséquent, que la composition tinctoriale selon l'invention permet d'obtenir des colorations plus résistantes aux shampooings.

### EXEMPLE 3- SYNTHESE

### Synthèse d'un composé de formule suivante :

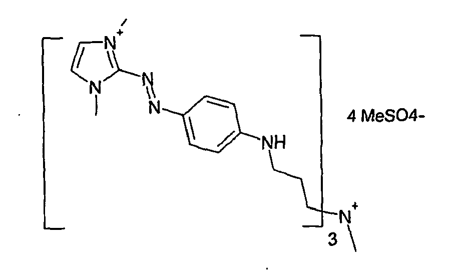

### Schéma réactionnel général:

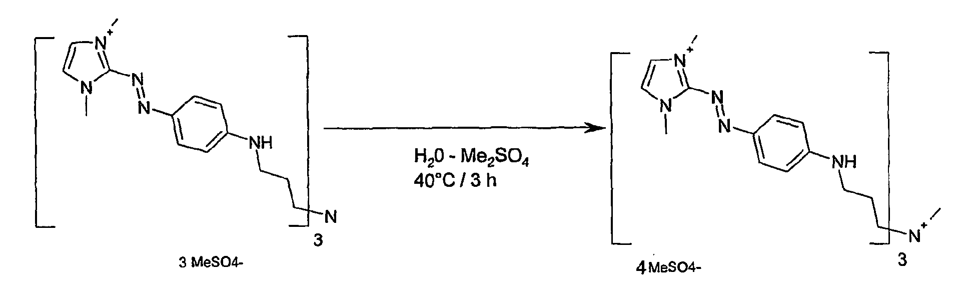

### Synthèse :

On dissout 0,5 g du colorant tricationique obtenu à l'exemple 1-1 dans 50 ml d'eau.

On ajoute ensuite 1 équivalent de sulfate de diméthyle.

Le mélange obtenu est conservé sous agitation à 40°C pendant 3 heures.

La solution résultante est versée dans 300 ml d'acétone. Le précipité est filtré,

lavé par 500 ml d'acétone pour obtenir le colorant tétracationique sous forme d'une poudre de couleur rouge.

Les analyses confirment la structure ci-dessus du composé obtenu.

On effectue la coloration d'une mèche conformément à la description faite dans l'exemple 2.

On obtient une mèche colorée en rouge.

### EXEMPLE 4 - SYNTHESE

### Synthèse d'un composé de formule suivante :

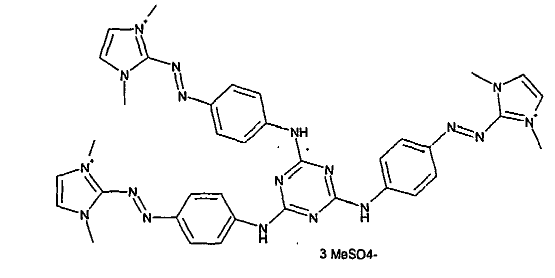

### Schéma réactionnel général :

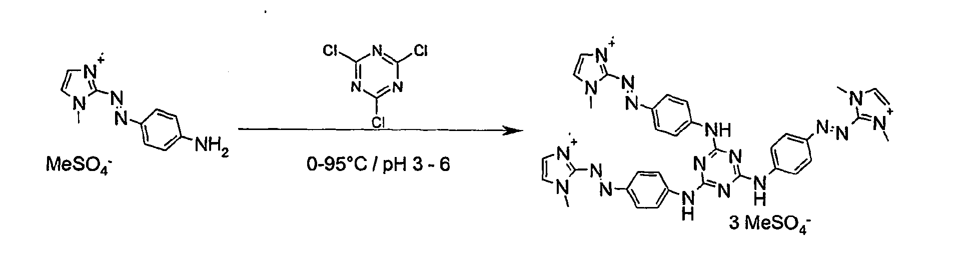

### Synthèse :

On dissout 0,5 g du colorant monoazoïque monocationique dans 20 ml d'eau.

On dissout 0,33 équivalent de trichlorotriazine dans 10 ml d'acétone. On verse la solution contenant la trichlorotriazine dans un mélange de glace (10 g) et d'eau (50 ml).

On ajoute ensuite la solution contenant le colorant monocationique.

Le mélange obtenu est conservé sous agitation à 0-5°C pendant 30 minutes.

Le mélange réactionnel est ensuite agité à 40°C pendant 30 minutes puis à 95°C pendant 2 heures.

Pendant la réaction on ajoute goutte à goutte une solution aqueuse saturée de NaHCO₃ pour maintenir le pH entre 3 et 6.

La solution résultante est refroidie à température ambiante et versée dans 300 ml d'acétone. Le précipité est filtré puis lavé par 500 ml d'acétone (500 ml) pour obtenir le colorant tricationique de couleur rouge.

Les analyses confirment la structure ci-dessus du composé obtenu.

On effectue la coloration d'une mèche conformément à la description faite dans l'exemple 2.

On obtient une mèche colorée en rouge.

## Revendications

1. Colorant direct cationique de formule (I) suivante : 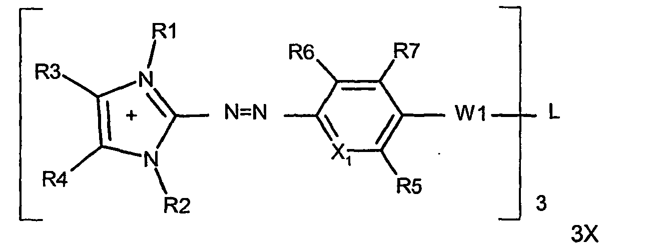 Formule dans laquelle
* W₁ représente -NR₈- ou -O- ;
* X₁ représente N ; CR₉;
• R₁, R₂, identiques ou différents, représentent une chaîne hydrocarbonée ;
• R₃, R₄ identiques ou différents, représentent un atome d'hydrogène, un atome d'halogène, un groupement nitro, un groupement cyano, une chaîne hydrocarbonée ;
• R₅, R₆, R₇, R₈, R₉, identiques ou différents, représentant un atome d'hydrogène, une chaîne hydrocarbonée ;
* L représente l'un des groupements ci-dessous : 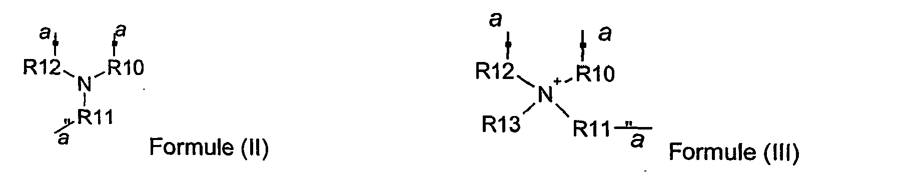 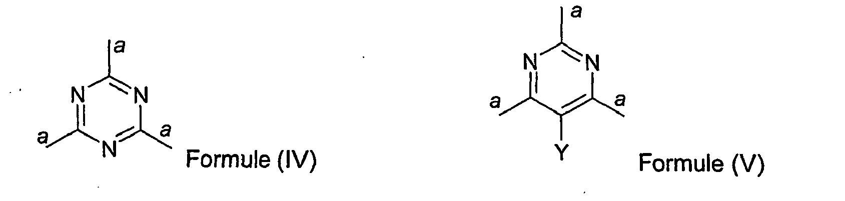 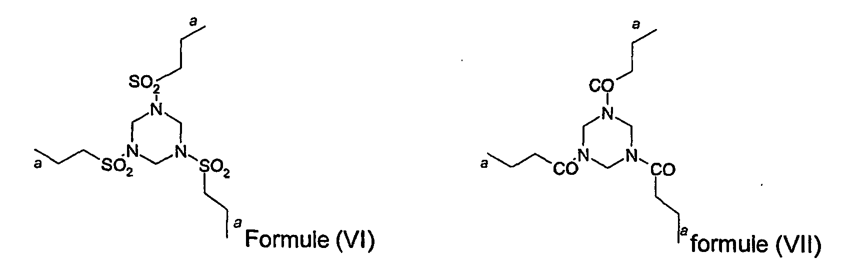 dans lesquelles :
- R₁₀, R₁₁, R₁₂, R₁₃, identiques ou différents, représentant une chaîne hydrocarbonée ;
- Y représente un atome d'halogène, de préférence le fluor ou le chlore ;
- la liaison a des formules (II) à (VII) est reliée au groupe W₁ de la formule (I);
* X représente un anion organique ou minéral cosmétiquement acceptable.

2. Colorant direct selon la revendication précédente, **caractérisé en ce que** dans les formules (I) à (VII), la chaîne hydrocarbonée est :
• linéaire ou ramifiée, saturée ou insaturée en C₁-C₈, éventuellement interrompue par un ou plusieurs hétéroatomes tels que l'oxygène, l'azote ou le soufre, et/ou par un ou plusieurs groupements carbonyle ou SO₂ ; ladite chaîne ne comprenant pas un hétéroatome adjacent à un ou plusieurs hétéroatomes, ni un groupement carbonyle ou SO₂ adjacent à un ou plusieurs groupements carbonyle et/ou SO₂ ;
• et/ou aromatique en C₅-C₆
• de plus la chaîne hydrocarbonée est éventuellement substituée par un ou plusieurs radicaux choisis parmi un radical hydroxyle ; un atome d'halogène, et de préférence le chlore, le fluor ; un radical alcoxy en C₁-C₄ ; un radical monohydroxy alcoxy dont la partie alkyle est en C₁-C₄, linéaire ou ramifiée, substituée ou non ; un radical polyhydroxy alcoxy, dans lequel la partie alkyle est en C₂-C₄, linéaire ou ramifiée, substituée ou non ; un radical amino substitué ou non par un ou plusieurs radicaux alkyle, identiques ou différents, en C₁-C₄, linéaires ou ramifiés, substitués ou non ; un radical thiol ; un radical alkylthio dont la partie alkyle est en C₁-C₄, linéaire ou ramifiée, substituée ou non ; un radical carboxylique ou forme acide ou salifiée (avec un métal alcalin ou un ammonium substitué ou non) ; un radical alcoxycarbonyle dont la partie alkyle est en C₁-C₄, linéaire ou ramifiée, substituée ou non ; un radical alkylamide dont la partie alkyle est en C₁-C₄, linéaire ou ramifiée, substituée ou non ; un radical alkylcarbamyle dont la partie alkyle est en C₁-C₄, linéaire ou ramifiée, substituée ou non ; un radical nitro ; un radical sulfonyle ; un radical alkylsulfonyle dont la partie alkyle est en C₁-C₄, linéaire ou ramifiée, substituée ou non ; un radical sulfonylamino, un radical alkylsulfonylamido dont la partie alkyle est en C₁-C₄, linéaire ou ramifiée, substituée ou non.

3. Colorant direct selon l'une quelconque des revendications précédentes, **caractérisé en ce que** les radicaux R₁, R₂, identiques ou différents, représentent un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₈ ; un radical C₆-aryl (C₁-C₄)alkyle.

4. Colorant direct selon la revendication précédente, **caractérisé en ce que** les radicaux R₁, R₂, identiques ou différents, représentent un radical alkyle en C₁-C₄, un radical benzyle.

5. Colorant direct selon la revendication précédente, **caractérisé en ce que** les radicaux R₁, R₂, identiques ou différents, représentent de préférence un radical méthyle, éthyle.

6. Colorant direct selon l'une quelconque des revendications précédentes, **caractérisé en ce que** les radicaux R₃, R₄, identiques ou différents, représentent un atome d'hydrogène ; un atome d'halogène ; un groupement nitro ; un groupement cyano ; un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteurs d'au moins un radical hydroxyle ; un radical alcoxy en C₁-C₈ ; un radical alkylthio en C₁-C₈ ; un radical sulfonylamino ; un radical phényle.

7. Colorant direct selon la revendication précédente, **caractérisé en ce que** les radicaux R₃, R₄, identiques ou différents, représentent un atome d'hydrogène ; un atome de chlore ; un radical alkyle en C₁-C₈ ou alcényle en C₂-C₈, éventuellement porteur d'un ou plusieurs radicaux hydroxyle, amino, amino substitué par un ou plusieurs radicaux alkyle en C₁-C₈ éventuellement porteur d'au moins un radical hydroxyle, un radical alcoxy en C₁-C₈ ; un radical phényle.

8. Colorant direct selon la revendication précédente, **caractérisé en ce que** les radicaux R₃, R₄, représentent un atome d'hydrogène, un atome de chlore, un radical phényle.

9. Colorant direct selon l'une quelconque des revendications précédentes, **caractérisé en ce que** les radicaux R₅, R₆, R₇, R₈, R₉, identiques ou différents, représentent un atome d'hydrogène ; un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₈ ; un radical alkylthio en C₁-C₈ ; un radical sulfonylamino.

10. Colorant direct selon la revendication précédente, **caractérisé en ce que** les radicaux R₅, R₆, R₇, R₈, R₉, identiques ou différents, représentent un atome d'hydrogène, un radical alkyle en C₁-C₄ éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₄.

11. Colorant direct selon la revendication précédente, **caractérisé en ce que** les radicaux R₅, R₆, R₇, R₈, R₉, identiques ou différents, représentent un atome d'hydrogène, un radical alkyle en C₁-C₄.

12. Colorant direct selon l'une quelconque des revendications précédentes, **caractérisé en ce que** les radicaux R₃, R₄, R₅, R₆, R₇, R₈, R₉, représentent un atome d'hydrogène.

13. Colorant direct selon l'une quelconque des revendications précédentes, **caractérisé en ce que** les radicaux R₁₀, R₁₁, R₁₂, R₁₃, identiques ou différents, représentent plus particulièrement un atome d'hydrogène ; un radical alkyle en C₁-C₈ ou alcényle en C₂-C₈, éventuellement porteur d'un ou plusieurs radicaux hydroxyle, amino, amino substitué par un ou plusieurs radicaux alkyles en C₁-C₈ éventuellement porteur d'au moins un radical hydroxyle ; un radical alcoxy en C₁-C₈ ; un radical alkylthio en C₁-C₈, un radical sulfonylamino.

14. Colorant direct selon la revendication précédente, **caractérisé en ce que** les radicaux R₁₀, R₁₁, R₁₂, R₁₃, selon un mode de réalisation préféré de l'invention, identiques ou différents, représentent un radical alkyle en C₁-C₄ éventuellement porteur d'un ou plusieurs radicaux hydroxyle, amino, amino substitué par un ou plusieurs radicaux alkyles C₁-C₄ éventuellement porteur d'au moins un radical hydroxyle ; un radical alcoxy en C₁-C₄.

15. Colorant direct selon la revendication précédente, **caractérisé en ce que** les radicaux R₁₀, R₁₁, R₁₂, R₁₃, identiques ou différents, représentent un radical éthyle, n-propyle, n-butyle.

16. Colorant direct selon l'une quelconque des revendications précédentes, **caractérisé en ce que** X₁ représente un groupement -CR₉-.

17. Colorant direct selon la revendication précédente, **caractérisé en ce que** R₉ représente un atome d'hydrogène.

18. Colorant direct selon l'une quelconque des revendications précédentes, **caractérisé en ce que** W₁ représente un groupement -NR₈-.

19. Colorant direct selon la revendication précédente, **caractérisé en ce que** R₈ représente un radical alkyle en C₁-C₄ et de préférence un atome d'hydrogène.

20. Colorant direct selon l'une quelconque des revendications précédentes, **caractérisé en ce que** le colorant direct de formule (I) comprend en outre un anion cosmétiquement acceptable, de nature organique ou minérale.

21. Colorant direct selon l'une quelconque des revendications précédentes, **caractérisé en ce que** X représente un anion minéral choisi parmi les halogénures, les hydroxydes, les sulfates, les hydrogénosulfates ; ou choisi parmi les anions organiques, comme l'acétate, le citrate, le tartrate, les alkylsulfates pour lesquels la partie alkyle, linéaire ou ramifiée, est en C₁-C₆, les alkylsulfonates pour lesquels la partie alkyle, linéaire ou ramifiée, est en C₁-C₆, les arylsulfonates pour lesquels la partie aryle, de préférence phényle, est éventuellement substituée par un ou plusieurs radicaux alkyle en C₁-C₄.

22. Colorant direct selon l'une quelconque des revendications précédentes, **caractérisé en ce que** le colorant est choisi parmi :
• Trichlorure de 2-((E)-{4-[(3-{bis[3-({4-[(E)-(1,3-dimethyl-1H-imidazol-3-ium-2-yl) diazényl]phényl}amino)propyl]amino}propyl)amino]phényl}diazényl)-1,3-diméthyl-1H-imidazol-3-ium
• Tétrachlorure de 2-{(E)-[4-({3-[bis[3-({4-[(E)-(1,3-diméthyl-1H-imidazol-3-ium-2-yl) diazényl]phényl}amino)propyl](méthyl)ammonio]propyl}amino)phényl]diazényl}-1,3-diméthyl-1H-imidazol-3-ium
• Tétrachlorure de 2-((E)-{4-[{3-[bis{3-[{4-[(E)-(1,3-diméthyl-1H-imidazol-3-ium-2-yl)diazényl]phényl}(méthyl)amino]propyl}(méthyl)ammonio]propyl}(méthyl)amino]phé nyl}diazényl)-1,3-diméthyl-1H-imidazol-3-ium
• Tétrachlorure de 2-((E)-{4-[{3-[bis{3-[{4-[(E)-(1,3-diéthyl-1H-imidazol-3-ium-2-yl)diazényl]phényl}(éthyl)amino]propyl}(éthyl)ammonio]propyl}(ethyl)amino]phényl}di azényl)-1,3-diéthyl-1H-imidazol-3-ium
• Tétrachlorure de 2-((E)-{4-[{3-[bis{3-[{4-[(E)-(1,3-diméthyl-1H-imidazol-3-ium-2-yl)diazényl]phényl}(éthyl)amino]propyl}(éthyl)ammonio]propyl}(éthyl)amino]phényl}di azényl)-1,3-diméthyl-1H-imidazol-3-ium
• Trichlorure de 2-((E)-{4-[(3-{3,5-bis[3-({4-[(E)-(1,3-diméthyl-1H-imidazol-3-ium-2-yl)diazényl]phényl}amino)propanoyl]-1,3,5-triazinan-1-yl}-3-oxopropyl)amino]phényl} diazényl)-1,3-diméthyl-1H-imidazol-3-ium
• Trichlorure de 2-((E)-{4-[[3-(3,5-bis{3-[{4-[(E)-(1,3-dimtéhyl-1H-imidazol-3-ium-2-yl)diazényl]phényl}(éthyl)amino]propanoyl}-1,3,5-triazinan-1-yl)-3-oxopropyl](éthyl) amino]phényl}diazényl)-1,3-diméthyl-1H-imidazol-3-ium
• Trichlorure de 2-{(E)-[4-({2-[(3,5-bis{[2-({4-[(E)-(1,3-diméthyl-1H-imidazol-3-ium-2-yl)diazényl]phényl}amino)éthyl]sulfonyl}-1,3,5-triazinan-1-yl) sulfonyl]éthyl}amino) phényl]diazényl}-1,3-diméthyl-1H-imidazol-3-ium
• Trichlorure de 2-((E)-{4-[(2-{[3,5-bis({2-[{4-[(E)-(1,3-diméthyl-1H-imidazol-3-ium-2-yl) diazényl]phényl}(éthyl)amino]éthyl}sulfonyl)-1,3,5-triazinan-1-yl]sulfonyl}éthyl)(éthyl) amino]phényl}diazényl)-1,3-dimethyl-1H-imidazol-3-ium
• Trichlorure de 2-[(E)-(4-{[4,6-bis({4-[(E)-(1,3-diméthyl-1H-imidazol-3-ium-2-yl) diazényl]phényl}amino)-1,3,5-triazin-2-yl]amino}phényl)diazényl]-1,3-diméthyl-1H-imidazol-3-ium
• Trichlorure de 2-((E)-{4-[{4,6-bis[{4-[(E)-(1,3-diméthyl-1H-imidazol-3-ium-2-yl) diazényl]phényl}(méthyl)amino]-1,3,5-triazin-2-yl}(méthyl)amino]phényl}diazényl)-1,3-diméthyl-1H-imidazol-3-ium
• Trichlorure de 2-[(E)-(4-{[5-chloro-2,6-bis({4-[(E)-(1,3-diméthyl-1H-imidazol-3-ium-2-yl)diazényl]phényl}amino)pyrimidin-4-yl]amino}phényl)diazényl]-1,3-diméthyl-1H-imidazol-3-ium
• Trichlorure de 2-((E)-{4-[{5-chloro-2,6-bis[{4-[(E)-(1,3-diméthyl-1H-imidazol-3-ium-2-yl)diazényl]phényl}(méthyl)amino]pyrimidin-4-yl}(méthyl)amino]phényl}diazényl)-1,3-diméthyl-1H-imidazol-3-ium ;
Ainsi que les composés dans lesquels le contre-ion est différent du chlore et choisi parmi les anions cosmétiquement acceptables, de nature organique ou minérale.

23. Composition tinctoriale comprenant dans un milieu approprié pour la coloration des fibres kératiniques humaines, au moins un colorant direct cationique de formule (I) selon l'une quelconque des revendications 1 à 22.

24. Composition tinctoriale selon la revendication précédente, **caractérisée en ce que** la teneur en colorant de formule (I) est comprise entre 0,001 et 20% en poids par rapport au poids de la composition tinctoriale, de préférence entre 0,01 et 10% en poids par rapport au poids de la composition tinctoriale.

25. Composition tinctoriale selon l'une quelconque des revendications 23 ou 24, **caractérisée en ce qu'**elle comprend au moins un colorant direct additionnel différent de celui de formule (I), cationique, anionique ou non ionique, avec une teneur comprise entre 0,001 et 20% en poids par rapport à la composition tinctoriale, et de préférence entre 0,01 et 10% du poids total de la composition tinctoriale.

26. Composition tinctoriale selon l'une quelconque des revendications 23 à 25, **caractérisée en ce qu'**elle comprend au moins une base d'oxydation avec une teneur comprise entre 0,0005 et 12% en poids par rapport au poids de la composition tinctoriale, avantageusement entre 0,005 et 8% en poids par rapport au poids de la composition tinctoriale.

27. Composition tinctoriale selon la revendication précédente, **caractérisée en ce qu'**elle comprend au moins un coupleur avec une teneur comprise entre 0,0001 et 10% en poids par rapport au poids de la composition tinctoriale, et de préférence entre 0,005 et 5% en poids par rapport au poids de la composition tinctoriale.

28. Composition tinctoriale selon l'une quelconque des revendications 23 à 27, **caractérisée en ce qu'**elle comprend au moins un agent oxydant.

29. Composition tinctoriale selon la revendication précédente, **caractérisée en ce que** l'agent oxydant est choisi parmi le peroxyde d'hydrogène, les peroxydes de métaux alcalins ou alcalino-terreux ; le peroxyde d'urée, les bromates ou ferricyanures de métaux alcalins ; les enzymes, seuls ou en mélanges.

30. Composition tinctoriale selon l'une quelconque des revendications 28 ou 29, **caractérisée en ce que** la teneur en agent oxydant est comprise entre 1 et 20 % en poids par rapport au poids de la composition tinctoriale, de préférence entre 1 et 12 % en poids par rapport au poids de la composition tinctoriale.

31. Procédé de coloration des fibres kératiniques humaines dans lequel on met en oeuvre les étapes suivantes :
a) on met en contact lesdites fibres, sèches ou humides, avec la composition selon l'une quelconque des revendications 23 à 29, pendant une durée suffisante pour le développement de la coloration,
b) on rince éventuellement les fibres,
c) éventuellement on lave les fibres et on les rince,
d) on sèche les fibres ou on les laisse sécher.

32. Dispositif à plusieurs compartiments pour la mise en oeuvre du procédé selon la revendication 31 dans lequel un premier compartiment contient une composition tinctoriale comprenant au moins un colorant de formule (I), et un deuxième compartiment contient un agent oxydant.

## Claims

1. Cationic direct dye of formula (I) below: 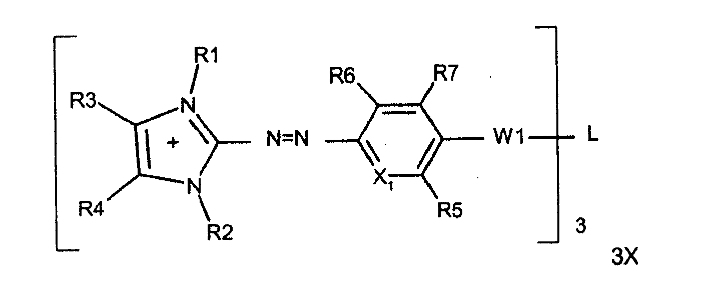 in which formula
* W₁ represents -NR₈- or -O-;
* X₁ represents N; CR₉;
• R₁ and R₂, which may be identical or different, represent a hydrocarbon chain;
• R₃ and R₄, which may be identical or different, represent a hydrogen atom, a halogen atom, a nitro group, a cyano group or a hydrocarbon chain;
• R₅, R₆, R₇, R₈ and R₉, which may be identical or different, represent a hydrogen atom or a hydrocarbon chain;
* L represents one of the groups below: 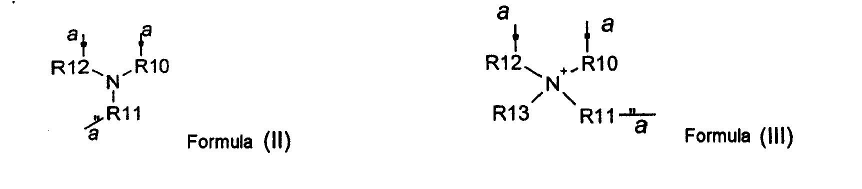 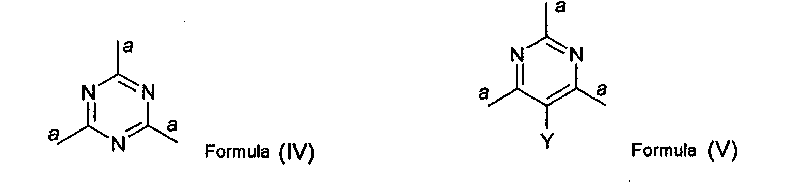 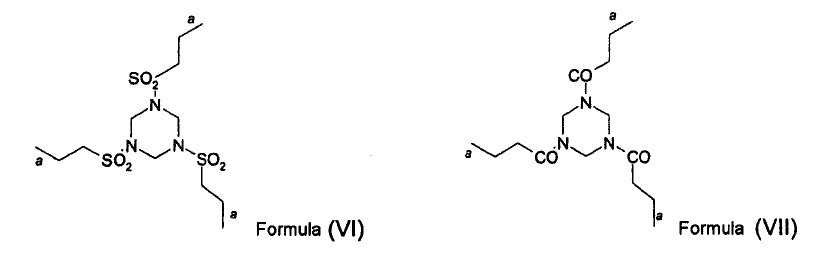 in which:
- R₁₀, R₁₁, R₁₂ and R₁₃, which may be identical or different, represent a hydrocarbon chain;
- Y represents a halogen atom, preferably fluorine or chlorine;
- the bond a of formulae (II) to (VII) is connected to the group W₁ of formula (I);
* X represents a cosmetically acceptable organic or mineral anion.

2. Direct dye according to the preceding claim, **characterized in that**, in formulae (I) to (VII), the hydrocarbon chain is:
• a linear or branched, saturated or unsaturated C₁-C₈ hydrocarbon chain optionally interrupted with one or more hetero atoms such as oxygen, nitrogen or sulphur, and/or with one or more carbonyl or SO₂ groups, said chain comprising no hetero atom adjacent to one or more hetero atoms, nor a carbonyl or SO₂ group adjacent to one or more carbonyl and/or SO₂ groups;
• and/or an aromatic C₅-C₆ hydrocarbon chain;
• furthermore, the hydrocarbon chain is optionally substituted with one or more radicals chosen from a hydroxyl radical; a halogen atom, and preferably chlorine or fluorine; a C₁-C₄ alkoxy radical; a monohydroxyalkoxy radical in which the alkyl portion is a linear or branched, substituted or unsubstituted C₁-C₄ alkyl; a polyhydroxyalkoxy radical, in which the alkyl portion is a linear or branched, substituted or unsubstituted C₂-C₄ alkyl; an amino radical that is unsubstituted or substituted with one or more linear or branched, substituted or unsubstituted C₁-C₄ alkyl radicals that may be identical or different; a thiol radical; an alkylthio radical in which the alkyl portion is a linear or branched, substituted or unsubstituted C₁-C₄ alkyl; a carboxyl radical either in acid form or salified form (with an alkali metal or an ammonium that may or may not be substituted); an alkoxycarbonyl radical in which the alkyl portion is a linear or branched, substituted or unsubstituted C₁-C₄ alkyl; an alkylamide radical in which the alkyl portion is a linear or branched, substituted or unsubstituted C₁-C₄ alkyl; an alkylcarbamyl radical in which the alkyl portion is a linear or branched, substituted or unsubstituted C₁-C₄ alkyl; a nitro radical; a sulphonyl radical; an alkylsulphonyl radical in which the alkyl portion is a linear or branched, substituted or unsubstituted C₁-C₄ alkyl; a sulphonylamino radical or an alkyl-sulphonylamido radical in which the alkyl portion is a linear or branched, substituted or unsubstituted C₁-C₄ alkyl.

3. Direct dye according to either one of the preceding claims, **characterized in that** the radicals R₁ and R₂, which may be identical or different, represent a C₁-C₈ alkyl or C₂-C₈ alkenyl radical optionally carrying one or more of the following radicals: hydroxyl, amino, amino substituted with one or more C₁-C₈ alkyl radicals optionally carrying at least one hydroxyl radical; a C₁-C₈ alkoxy radical; a C₆-aryl (C₁-C₄) alkyl radical.

4. Direct dye according to the preceding claim, **characterized in that** the radicals R₁ and R₂, which may be identical or different, represent a C₁-C₄ alkyl radical or a benzyl radical.

5. Direct dye according to the preceding claim, **characterized in that** the radicals R₁ and R₂, which may be identical or different, preferably represent a methyl or ethyl radical.

6. Direct dye according to any one of the preceding claims, **characterized in that** the radicals R₃ and R₄, which may be identical or different, represent a hydrogen atom; a halogen atom; a nitro group; a cyano group; a C₁-C₈ alkyl or C₂-C₈ alkenyl radical optionally carrying one or more of the following radicals: hydroxyl, amino, amino substituted with one or more C₁-C₈ alkyl radicals optionally carrying at least one hydroxyl radical; a C₁-C₈ alkoxy radical; a C₁-C₈ alkylthio radical; a sulphonylamino radical; a phenyl radical.

7. Direct dye according to the preceding claim, **characterized in that** the radicals R₃ and R₄, which may be identical or different, represent a hydrogen atom; a chlorine atom; a C₁-C₈ alkyl or C₂-C₈ alkenyl radical optionally carrying one or more of the following radicals: hydroxyl, amino, amino substituted with one or more C₁-C₈ alkyl radicals optionally carrying at least one hydroxyl radical, a C₁-C₈ alkoxy radical; a phenyl radical.

8. Direct dye according to the preceding claim, **characterized in that** the radicals R₃ and R₄ represent a hydrogen atom, a chlorine atom or a phenyl radical.

9. Direct dye according to any one of the preceding claims, **characterized in that** the radicals R₅, R₆, R₇, R₈ and R₉, which may be identical or different, represent a hydrogen atom; a C₁-C₈ alkyl or C₂-C₈ alkenyl radical optionally carrying one or more of the following radicals: hydroxyl, amino, amino substituted with one or more C₁-C₈ alkyl radicals optionally carrying at least one hydroxyl radical; a C₁-C₈ alkoxy radical; a C₁-C₈ alkylthio radical; a sulphonylamino radical.

10. Direct dye according to the preceding claim, **characterized in that** the radicals R₅, R₆, R₇, R₈ and R₉, which may be identical or different, represent a hydrogen atom, a C₁-C₄ alkyl radical optionally carrying one or more of the following radicals: hydroxyl, amino, amino substituted with one or more C₁-C₈ alkyl radicals optionally carrying at least one hydroxyl radical; a C₁-C₄ alkoxy radical.

11. Direct dye according to the preceding claim, **characterized in that** the radicals R₅, R₆, R₇, R₈ and R₉, which may be identical or different, represent a hydrogen atom or a C₁-C₄ alkyl radical.

12. Direct dye according to any one of the preceding claims, **characterized in that** the radicals R₃, R₄, R₅, R₆, R₇, R₈ and R₉ represent a hydrogen atom.

13. Direct dye according to any one of the preceding claims, **characterized in that** the radicals R₁₀, R₁₁, R₁₂ and R₁₃, which may be identical or different, represent more particularly a hydrogen atom; a C₁-C₈ alkyl or C₂-C₈ alkenyl radical optionally carrying one or more of the following radicals: hydroxyl, amino, amino substituted with one or more C₁-C₈ alkyl radicals optionally carrying at least one hydroxyl radical; a C₁-C₈ alkoxy radical; a C₁-C₈ alkylthio radical; a sulphonylamino radical.

14. Direct dye according to the preceding claim, **characterized in that** the radicals R₁₀, R₁₁, R₁₂ and R₁₃, according to a preferred embodiment of the invention, which may be identical or different, represent a C₁-C₄ alkyl radical optionally carrying one or more of the following radicals: hydroxyl, amino, amino substituted with one or more C₁-C₄ alkyl radicals optionally carrying at least one hydroxyl radical; a C₁-C₄ alkoxy radical.

15. Direct dye according to the preceding claim, **characterized in that** the radicals R₁₀, R₁₁, R₁₂ and R₁₃, which may be identical or different, represent an ethyl, n-propyl or n-butyl radical.

16. Direct dye according to any one of the preceding claims, **characterized in that** X₁ represents a group -CR₉- .

17. Direct dye according to the preceding claim, **characterized in that** R₉ represents a hydrogen atom.

18. Direct dye according to any one of the preceding claims, **characterized in that** W₁ represents a group -NR₈-.

19. Direct dye according to the preceding claim, **characterized in that** R₈ represents a C₁-C₄ alkyl radical, and preferably a hydrogen atom.

20. Direct dye according to any one of the preceding claims, **characterized in that** the direct dye of formula (I) also comprises a cosmetically acceptable anion that is organic or mineral in nature.

21. Direct dye according to any one of the preceding claims, **characterized in that** X represents a mineral anion chosen from halides, hydroxides, sulphates, hydrogen sulphates; or is chosen from organic anions, such as acetate, citrate, tartrate, alkyl sulphates for which the linear or branched alkyl portion is a C₁-C₆ alkyl, alkylsulphonates for which the linear or branched alkyl portion is a C₁-C₆ alkyl, arylsulphonates for which the aryl, preferably phenyl, portion is optionally substituted with one or more C₁-C₄ alkyl radicals.

22. Direct dye according to any one of the preceding claims, **characterized in that** the dye is chosen from:
• 2-((E)-{4-[(3-{bis[3-({4-[(E)-(1,3-dimethyl-1H-imidazol-3-ium-2-yl)diazenyl]phenyl}amino)-propyl]amino}propyl)amino]phenyl}diazenyl)-1,3-dimethyl-1H-imidazol-3-ium trichloride
• 2-{(E)-[4-({3-[bis[3-({4-[(E)-(1,3-dimethyl-1H-imidazol-3-ium-2-yl)diazenyl]phenyl}amino)-propyl](methyl)ammonio]propyl}amino)phenyl]-diazenyl}-1,3-dimethyl-1H-imidazol-3-ium tetrachloride
• 2-((E)-{4-[{3-[bis{3-[{4-[(E)-(1,3-dimethyl-1H-imidazol-3-ium-2-yl)diazenyl]phenyl}(methyl)-amino]propyl}(methyl)ammonio]propyl}(methyl)-amino]phenyl}diazenyl)-1,3-dimethyl-1H-imidazol-3-ium tetrachloride
• 2-((E)-{4-[{3-[bis{3-[{4-[(E)-(1,3-diethyl-1H-imidazol-3-ium-2-yl)diazenyl]phenyl}(ethyl)-amino]propyl}(ethyl)ammonio]propyl}(ethyl)-amino]phenyl}diazenyl)-1,3-diethyl-1H-imidazol-3-ium tetrachloride
• 2-((E)-{4-[{3-[bis{3-[{4-[(E)-(1,3-dimethyl-1H-imidazol-3-ium-2-yl)diazenyl]phenyl}(ethyl)-amino]propyl}(ethyl)ammonio]propyl}(ethyl)-amino]phenyl}diazenyl)-1,3-dimethyl-1H-imidazol-3-ium tetrachloride
• 2-((E)-{4-[(3-{3,5-bis[3-({4-[(E)-(1,3-dimethyl-1H-imidazol-3-ium-2-yl)diazenyl]phenyl}amino)-propanoyl]-1,3,5-triazinan-1-yl}-3-oxopropyl)-amino]phenyl}diazenyl)-1,3-dimethyl-1H-imidazol-3-ium trichloride
• 2-((E)-{4-[[3-(3,5-bis{3-[{4-[(E)-(1,3-dimethyl-1H-imidazol-3-ium-2-yl)diazenyl]phenyl}(ethyl)-amino]propanoyl}-1,3,5-triazinan-1-yl)-3-oxopropyl](ethyl)amino]phenyl}diazenyl)-1,3-dimethyl-1H-imidazol-3-ium trichloride
• 2-{(E)-[4-({2-[(3,5-bis{[2-({4-[(E)-(1,3-dimethyl-1H-imidazol-3-ium-2-yl)diazenyl]phenyl}-amino)ethyl]sulphonyl}-1,3,5-triazinan-1-yl)-sulphonyl]ethyl}amino)phenyl]diazenyl}-1,3-dimethyl-1H-imidazol-3-ium trichloride
• 2-((E)-{4-[(2-{[3,5-bis({2-[{4-[(E)-(1,3-dimethyl-1H-imidazol-3-ium-2-yl)diazenyl]phenyl}-(ethyl)amino]ethyl}sulphonyl)-1,3,5-triazinan-1-yl]sulphonyl}ethyl)(ethyl)amino]phenyl}-diazenyl)-1,3-dimethyl-1H-imidazol-3-ium trichloride
• 2-[(E)-(4-{[4,6-bis({4-[(E)-(1,3-dimethyl-1H-imidazol-3-ium-2-yl)diazenyl]phenyl}amino)-1,3,5-triazin-2-yl]amino}phenyl)diazenyl]-1,3-dimethyl-1H-imidazol-3-ium trichloride
• 2-((E)-{4-[{4,6-bis[{4-[(E)-(1,3-dimethyl-1H-imidazol-3-ium-2-yl)diazenyl]phenyl}(methyl)-amino]-1,3,5-triazin-2-yl}(methyl)amino]-phenyl}diazenyl)-1,3-dimethyl-1H-imidazol-3-ium trichloride
• 2-[(E)-(4-{[5-chloro-2,6-bis({4-[(E)-(1,3-dimethyl-1H-imidazol-3-ium-2-yl)diazenyl]phenyl}-amino)pyrimidin-4-yl]amino}phenyl)diazenyl]-1,3-dimethyl-1H-imidazol-3-ium trichloride
• 2-((E)-{4-[{5-chloro-2,6-bis[{4-[(E)-(1,3-dimethyl-1H-imidazol-3-ium-2-yl)diazenyl]phenyl}-(methyl)amino]pyrimidin-4-yl}(methyl)amino]-phenyl}diazenyl)-1,3-dimethyl-1H-imidazol-3-ium trichloride;
and also the compounds in which the counterion is different from chlorine and is chosen from cosmetically acceptable anions that are organic or mineral in nature.

23. Dye composition comprising, in a medium suitable for dyeing human keratin fibres, at least one cationic direct dye of formula (I) according to any one of Claims 1 to 22.

24. Dye composition according to the preceding claim, **characterized in that** the content of dye of formula (I) is between 0.001 and 20% by weight relative to the weight of the dye composition, preferably between 0.01 and 10% by weight relative to the weight of the dye composition.

25. Dye composition according to either one of Claims 23 and 24, **characterized in that** it comprises at least one additional cationic, anionic or nonionic direct dye that is different from that of formula (I), with a content of between 0.001 and 20% by weight relative to the dye composition, and preferably between 0.01 and 10% of the total weight of the dye composition.

26. Dye composition according to any one of Claims 23 to 25, **characterized in that** it comprises at least one oxidation base with a content of between 0.0005 and 12% by weight relative to the weight of the dye composition, advantageously between 0.005 and 8% by weight relative to the weight of the dye composition.

27. Dye composition according to the preceding claim, **characterized in that** it comprises at least one coupler with a content of between 0.0001 and 10% by weight relative to the weight of the dye composition, and preferably between 0.005 and 5% by weight relative to the weight of the dye composition.

28. Dye composition according to any one of Claims 23 to 27, **characterized in that** it comprises at least one oxidizing agent.

29. Dye composition according to the preceding claim, **characterized in that** the oxidizing agent is chosen from hydrogen peroxide, alkali metal peroxides or alkaline-earth metal peroxides; urea peroxide, alkali metal bromates or alkali metal ferricyanides; enzymes, alone or as mixtures.

30. Dye composition according to either one of Claims 28 and 29, **characterized in that** the content of oxidizing agent is between 1 and 20% by weight relative to the weight of the dye composition, preferably between 1 and 12% by weight relative to the weight of the dye composition.

31. Process for dyeing human keratin fibres, in which the following steps are carried out:
a) said fibres, dry or wet, are brought into contact with the composition according to any one of Claims 23 to 29 for a period of time sufficient to develop the coloration,
b) the fibres are optionally rinsed,
c) the fibres are optionally washed and rinsed,
d) the fibres are dried or are left to dry.

32. Multicompartment device for implementing the process according to Claim 31, in which a first compartment contains a dye composition comprising at least one dye of formula (I), and a second compartment contains an oxidizing agent.

## Patentansprüche

1. Kationischer Direktfarbstoff der folgenden Formel (I): 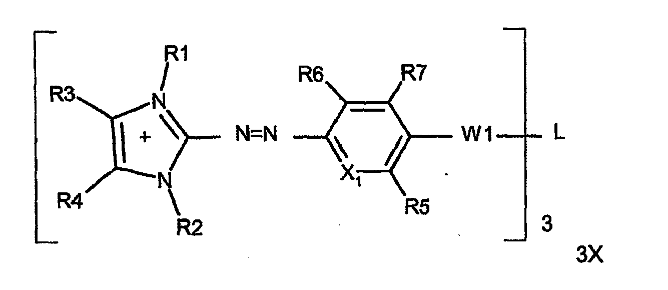 in der Formel:
* W₁ bedeutet -NR₈- oder -O-;
* X₁ bedeutet N; CR₉;
• die Gruppen R₁, R₂, die gleich oder verschieden sind, bedeuten eine Kohlenwasserstoffkette;
• die Gruppen R₃, R₄, die gleich oder verschieden sind, bedeuten ein Wasserstoffatom, ein Halogenatom, eine Nitrogruppe, eine Cyanogruppe, eine Kohlenwasserstoffkette;
• die Gruppen R₅, R₆, R₇, R₈, R₉, die gleich oder verschieden sind, bedeuten ein Wasserstoffatom, eine Kohlenwasserstoffkette;
* L bedeutet eine der folgenden Gruppen: 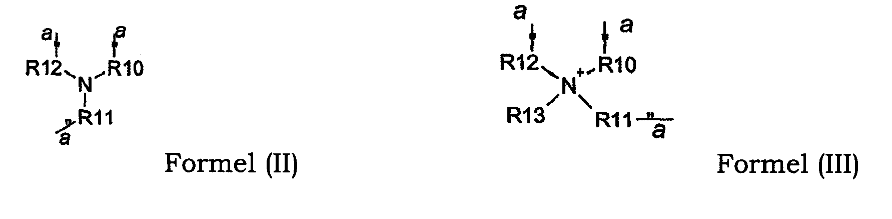 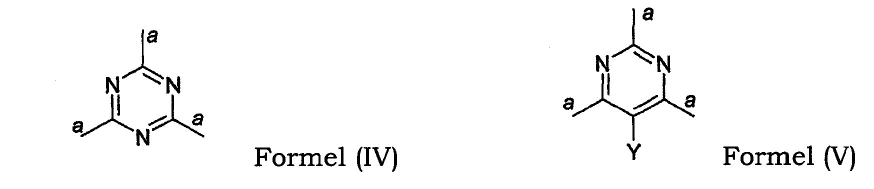 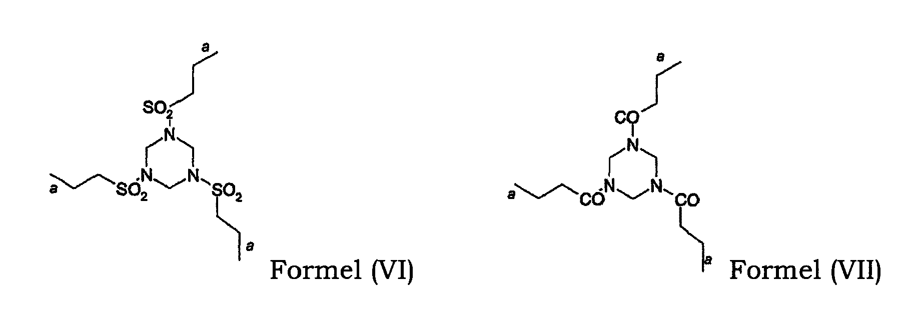 worin bedeuten:
- die Gruppen R₁₀, R₁₁, R₁₂, R₁₃, die gleich oder verschieden sind, eine Kohlenwasserstoffkette;
- Y ein Halogenatom, vorzugsweise Fluor oder Chlor,
- die Bindung a in den Formeln (II) bis (IV) ist mit der Gruppe W₁ der Formel (I) verbunden;
* X bedeutet ein kosmetisch akzeptables anorganisches oder organisches Anion.

2. Direktfarbstoff nach dem vorhergehenden Anspruch, **dadurch gekennzeichnet, dass** in den Formeln (I) bis (VII) die Kohlenwasserstoffkette ist:
• linear oder verzweigt, gesättigt oder ungesättigt mit 1 bis 8 Kohlenstoffatomen, wobei sie gegebenenfalls durch ein oder mehrere Heteroatome, wie Sauerstoff, Stickstoff oder Schwefel, und/oder durch eine oder mehrere Gruppen Carbonyl oder SO₂ unterbrochen ist; wobei die Kette kein Heteroatom, das einem oder mehreren Heteroatomen benachbart ist, und keine Gruppe Carbonyl oder SO₂ enthält, die einer oder mehreren Gruppen Carbonyl und/oder SO₂ benachbart ist;
• und/oder aromatisch mit 5 bis 6 Kohlenstoffatomen;
• ferner ist die Kohlenwasserstoffkette gegebenenfalls mit einer oder mehreren Gruppen substituiert, die ausgewählt sind unter einer Hydroxygruppe; einem Halogenatom und vorzugsweise Chlor, Fluor; einer C₁₋₄-Alkoxygruppe; einer Monohydroxyalkoxygruppe, deren C₁₋₄-Alkylteil linear oder verzweigt, substituiert oder unsubstituiert ist; einer Polyhydroxyalkoxygruppe, deren C₂₋₄-Alkylteil linear oder verzweigt, substituiert oder unsubstituiert ist; einer Aminogruppe, die unsubstituiert vorliegt oder mit einer oder mehreren, linearen oder verzweigten, substituierten oder unsubstituierten, gleichen oder verschiedenen C₁₋₄-Alkylgruppen substituiert ist; einer Thiogruppe; einer Alkylthiogruppe, deren C₁₋₄-Alkylteil linear oder verzweigt, substituiert oder unsubstituiert ist; einer Carboxygruppe in der Form der Säure oder als Salz (mit einem Alkalimetall oder einer Ammoniumgruppe, die substituiert oder unsubstituiert ist); einer Alkoxycarbonylgruppe, deren C₁₋₄-Alkylteil linear oder verzweigt, substituiert oder unsubstituiert ist; einer Alkylamidgruppe, deren C₁₋₄-Alkylteil linear oder verzweigt, substituiert oder unsubstituiert ist; einer Alkylcarbamoylgruppe, deren C₁₋₄-Alkylteil linear oder verzweigt, substituiert oder unsubstituiert ist; einer Nitrogruppe, einer Sulfonylgruppe; einer Alkylsulfonylgruppe, deren C₁₋₄-Alkylteil linear oder verzweigt, substituiert oder unsubstituiert ist; einer Sulfonylaminogruppe; einer Alkylsulfonylamidogruppe, deren C₁₋₄-Alkylteil linear oder verzweigt, substituiert oder unsubstituiert ist.

3. Direktfarbstoff nach einem der vorhergehenden Ansprüche, **dadurch gekennzeichnet, dass** die Gruppen R₁, R₂, die gleich oder verschieden sind, eine C₁₋₈-Alkylgruppe oder C₂₋₈-Alkenylgruppe bedeuten, die gegebenenfalls eine oder mehrere Hydroxygruppen, Aminogruppen oder Aminogruppen tragen, die mit einer oder mehreren C₁₋₈-Alkylgruppen substituiert sind, die gegebenenfalls mindestens eine Hydroxygruppe aufweisen; eine C₁₋₈-Alkoxygruppe; eine C₆-Aryl-(C₁₋₄)alkylgruppe bedeuten.

4. Direktfarbstoff nach dem vorhergehenden Anspruch, **dadurch gekennzeichnet, dass** die Gruppen R₁, R₂, die gleich oder verschieden sind, eine C₁₋₄-Alkylgruppe, eine Benzylgruppe bedeuten.

5. Direktfarbstoff nach dem vorhergehenden Anspruch, **dadurch gekennzeichnet, dass** die Gruppen R₁, R₂, die gleich oder verschieden sind, vorzugsweise Methyl, Ethyl bedeuten.

6. Direktfarbstoff nach einem der vorhergehenden Ansprüche, **dadurch gekennzeichnet, dass** die Gruppen R₃, R₄, die gleich oder verschieden sind, ein Wasserstoffatom; ein Halogenatom; eine Nitrogruppe; eine Cyanogruppe; eine C₁₋₈-Alkylgruppe oder C₂₋₈-Alkenylgruppe, die gegebenenfalls eine oder mehrere Hydroxygruppen, Aminogruppen oder Aminogruppen tragen, die mit einer oder mehreren C₁₋₈-Alkylgruppen substituiert sind, die gegebenenfalls mindestens eine Hydroxygruppe aufweisen; eine C₁₋₈-Alkoxygruppe; eine C₁₋₈-Alkylthiogruppe; eine Sulfonylaminogruppe; eine Phenylgruppe bedeuten.

7. Direktfarbstoff nach dem vorhergehenden Anspruch, **dadurch gekennzeichnet, dass** die Gruppen R₃, R₄, die gleich oder verschieden sind, ein Wasserstoffatom; ein Chloratom; eine C₁₋₈-Alkylgruppe oder C₂₋₈-Alkenylgruppe, die gegebenenfalls eine oder mehrere Hydroxygruppen, Aminogruppen oder Aminogruppen tragen, die mit einer oder mehreren C₁₋₈-Alkylgruppen substituiert sind, die gegebenenfalls mindestens eine Hydroxygruppe aufweisen; eine C₁₋₈-Alkoxygruppe; eine Phenylgruppe bedeuten.

8. Direktfarbstoff nach dem vorhergehenden Anspruch, **dadurch gekennzeichnet, dass** die Gruppen R₃, R₄ ein Wasserstoffatom; ein Chloratom; eine Phenylgruppe bedeuten.

9. Direktfarbstoff nach einem der vorhergehenden Ansprüche, **dadurch gekennzeichnet, dass** die Gruppen R₅, R₆, R₇, R₈, R₉, die gleich oder verschieden sind, ein Wasserstoffatom; eine C₁₋₈-Alkylgruppe oder C₂₋₈-Alkenylgruppe, die gegebenenfalls eine oder mehrere Hydroxygruppen, Aminogruppen oder Aminogruppen tragen, die mit einer oder mehreren C₁₋₈-Alkylgruppen substituiert sind, die gegebenenfalls mindestens eine Hydroxygruppe aufweisen; eine C₁₋₈-Alkoxygruppe; C₁₋₈-Alkylthiogruppe; eine Sulfonylaminogruppe bedeuten.

10. Direktfarbstoff nach dem vorhergehenden Anspruch, **dadurch gekennzeichnet, dass** die Gruppen R₅, R₆, R₇, R₈, R₉, die gleich oder verschieden sind, ein Wasserstoffatom; eine C₁₋₄-Alkylgruppe, die gegebenenfalls eine oder mehrere Hydroxygruppen, Aminogruppen, Aminogruppen trägt, die mit einer oder mehreren C₁₋₈-Alkylgruppen substituiert sind, die gegebenenfalls mindestens eine Hydroxygruppe aufweisen; eine C₁₋₄-Alkoxygruppe bedeuten.

11. Direktfarbstoff nach dem vorhergehenden Anspruch, **dadurch gekennzeichnet, dass** die Gruppen R₅, R₆, R₇, R₈, R₉, die gleich oder verschieden sind, ein Wasserstoffatom; eine C₁₋₄-Alkylgruppe bedeuten.

12. Direktfarbstoff nach einem der vorhergehenden Ansprüche, **dadurch gekennzeichnet, dass** die Gruppen R₃, R₄, R₅, R₆, R₇, R₈, R₉ ein Wasserstoffatom bedeuten.

13. Direktfarbstoff nach einem der vorhergehenden Ansprüche, **dadurch gekennzeichnet, dass** die Gruppen R₁₀, R₁₁, R₁₂, R₁₃, die gleich oder verschieden sind, insbesondere ein Wasserstoffatom; eine C₁₋₈-Alkylgruppe oder C₂₋₈-Alkenylgruppe, die gegebenenfalls eine oder mehrere Hydroxygruppen, Aminogruppen oder Aminogruppen tragen, die mit einer oder mehreren C₁₋₈-Alkylgruppen substituiert sind, die gegebenenfalls mindestens eine Hydroxygruppe aufweisen; eine C₁₋₈-Alkoxygruppe; eine C₁₋₈-Alkylthiogruppe; eine Sulfonylaminogruppe bedeuten.

14. Direktfarbstoff nach dem vorhergehenden Anspruch, **dadurch gekennzeichnet, dass** die Gruppen R₁₀, R₁₁, R₁₂, R₁₃, die gleich oder verschieden sind, eine C₁₋₄-Alkylgruppe, die gegebenenfalls eine oder mehrere Hydroxygruppen, Aminogruppen oder Aminogruppen trägt, die mit einer oder mehreren C₁₋₈-Alkylgruppen substituiert sind, die gegebenenfalls mindestens eine Hydroxygruppe aufweisen; eine C₁₋₄-Alkoxygruppe bedeuten.

15. Direktfarbstoff nach dem vorhergehenden Anspruch, **dadurch gekennzeichnet, dass** die Gruppen R₁₀, R₁₁, R₁₂, R₁₃, die gleich oder verschieden sind, Ethyl, *n*-Propyl, *n*-Butyl bedeuten.

16. Direktfarbstoff nach einem der vorhergehenden Ansprüche, **dadurch gekennzeichnet, dass** die Gruppe X₁ eine Gruppe -CR₉- ist.

17. Direktfarbstoff nach dem vorhergehenden Anspruch, **dadurch gekennzeichnet, dass** R₉ ein Wasserstoffatom bedeutet.

18. Direktfarbstoff nach einem der vorhergehenden Ansprüche, **dadurch gekennzeichnet, dass** die Gruppe W₁ eine Gruppe -NR₈- ist.

19. Direktfarbstoff nach dem vorhergehenden Anspruch, **dadurch gekennzeichnet, dass** R₈ eine C₁₋₄-Alkylgruppe und vorzugsweise ein Wasserstoffatom bedeutet.

20. Direktfarbstoff nach einem der vorhergehenden Ansprüche, **dadurch gekennzeichnet, dass** der Direktfarbstoff der Formel (I) ferner ein kosmetisch akzeptables Anion vom organischen oder anorganischen Typ umfasst.

21. Direktfarbstoff nach einem der vorhergehenden Ansprüche, **dadurch gekennzeichnet, dass** X ein anorganisches Anion bedeutet, das unter den Halogeniden, Hydroxiden, Sulfaten, Hydrogensulfaten ausgewählt ist; oder unter den organisches Anionen ausgewählt ist, wie Acetat, Citrat, Tartrat, Alkylsulfaten, bei denen der lineare oder verzweigte Alkylteil 1 bis 6 Kohlenstoffatome aufweist, Alkylsulfonaten, bei denen der lineare oder verzweigte Alkylteil 1 bis 6 Kohlenstoffatome aufweist, Arylsulfonaten, bei denen der Arylteil und vorzugsweise Phenylteil gegebenenfalls mit einer oder mehreren C₁₋₄-Alkylgruppen substituiert ist.

22. Direktfarbstoff nach einem der vorhergehenden Ansprüche, **dadurch gekennzeichnet, dass** der Farbstoff ausgewählt ist unter:
• 2-((E)-{4-[(3-{Bis[3-({4-[(E)-(1,3-dimethyl-1H-imidazol-3-ium-2-yl)diazenyl]phenyl}amino)propyl]amino}propyl)amino]phenyl}-diazenyl)-1,3-dimethyl-1 H-imidazol-3-ium-trichlorid;
• 2-{(E)-[4-({3-[Bis[3-({4-[(E)-(1,3-dimethyl-1H-imidazol-3-ium-2-yl)diazenyl]phenyl}amino)propyl] (methyl)ammonio]propyl}amino)-phenyl]diazenyl}-1,3-dimethyl-1H-imidazol-3-ium-tetrachlorid;
• 2-((E)-{4-[{3-[Bis{3-[{4-[(E)-(1,3-dimethyl-1H-imidazol-3-ium-2-yl)diazenyl]phenyl}(methyl)amino]propyl}(methyl)ammonio]propyl}-(methyl)amino]phenyl}diazenyl)-1,3-dimethyl-1H-imidazol-3-ium-tetrachlorid;
• 2-((E)-{4-[{3-[Bis{3-[{4-[(E)-(1,3-diethyl-1H-imidazol-3-ium-2-yl)diazenyl]phenyl}(ethyl)amino]propyl}(ethyl)ammonio]propyl}-(ethyl)amino]phenyl}diazenyl)-1,3-diethyl-1 H-imidazol-3-ium-tetrachlorid;
• 2-((E)-{4-[{3-[Bis{3-[{4-[(E)-(1,3-dimethyl-1H-imidazol-3-ium-2-yl)diazenyl]phenyl}(ethyl)amino]propyl}(ethyl)ammonio]propyl}-(ethyl)amino]phenyl}diazenyl)-1,3-dimethyl-1H-imidazol-3-ium-tetrachlorid;
• 2-((E)-{4-[(3-{3,5-Bis[3-({4-[(E)-(1,3-dimethyl-1H-imidazol-3-ium-2-yl)diazenyl]phenyl}amino)propanoyl]-1,3,5-triazinan-1-yl}-3-oxopropyl)amino]phenyl}diazenyl)-1,3-dimethyl-1 H-imidazol-3-ium-trichlorid;
• 2-((E)-{4-[[3-(3,5-Bis{3-[{4-[(E)-(1,3-dimethyl-1H-imidazol-3-ium-2-yl)diazenyl]phenyl}(ethyl)amino]propanoyl}-1,3,5-triazinan-1-yl)-3-oxopropyl](ethyl)amino]phenyl}diazenyl)-1,3-dimethyl-1 H-imidazol-3-ium-trichlorid;
• 2-{(E)-[4-({2-[(3,5-Bis{[2-({4-[(E)-(1,3-dimethyl-1H-imidazol-3-ium-2-yl)diazenyl]phenyl}amino)ethyl]sulfonyl}-1,3, 5-triazinan-1-yl)-sulfonyl]ethyl}amino) phenyl]diazenyl}-1,3-dimethyl-1H-imidazol-3-ium-trichlorid;
• 2-((E)-{4-[(2-{[3,5-Bis({2-[{4-[(E)-(1,3-dimethyl-1H-imidazol-3-ium-2-yl)diazenyl]phenyl}(ethyl)amino]ethyl}sulfonyl)-1,3,5-triazinan-1-yl]sulfonyl}ethyl)(ethyl)amino]phenyl}diazenyl)-1,3-dimethyl-1H-imidazol-3-ium-trichlorid;
• 2-[(E)-(4-{[4,6-Bis({4-[(E)-(1,3-dimethyl-1H-imidazol-3-ium-2-yl)di-azenyl]phenyl}amino)-1,3,5-triazin-2-yl]amino}phenyl)diazenyl]-1,3-dimethyl-1 H-imidazol-3-ium-trichlorid;
• 2-((E)-{4-[{4,6-Bis[{4-[(E)-(1,3-dimethyl-1H-imidazol-3-ium-2-yl)di-azenyl]phenyl}(methyl)amino]-1,3,5-triazin-2-yl}(methyl)amino] phenyl}diazenyl)-1,3-dimethyl-1H-imidazol-3-ium-trichlorid;
• 2-[(E)-(4-{[5-Chlor-2,6-bis({4-[(E)-(1,3-dimethyl-1H-imidazol-3-ium-2-yl)diazenyl]phenyl}amino)pyrimidin-4-yl]amino}phenyl)diazenyl]-1, 3-dimethyl-1 H-imidazol-3-ium-trichlorid;
• 2-((E)-{4-[{5-Chlor-2,6-bis[{4-[(E)-(1,3-dimethyl-1H-imidazol-3-ium-2-yl)diazenyl]phenyl}(methyl)amino]pyrimidin-4-yl}(methyl)amino]-phenyl}diazenyl)-1,3-dimethyl-1H-imidazol-3-ium-trichlorid;
• sowie den Verbindungen, bei denen das Gegenion von Chlor verschieden und unter den kosmetisch akzeptablen Anionen vom organischen oder anorganischen Typ ausgewählt ist.

23. Farbmittelzusammensetzung, die in einem zum Färben von menschlichen Keratinfasern geeigneten Medium mindestens einen kationischen Direktfarbstoff der Formel (I) nach einem der Ansprüche 1 bis 22 enthält.

24. Farbmittelzusammensetzung nach dem vorhergehenden Anspruch, **dadurch gekennzeichnet, dass** der Mengenanteil des Farbstoffes der Formel (I) im Bereich von 0,001 bis 20 Gew.-%, bezogen auf das Gewicht der Farbmittelzusammensetzung, und vorzugsweise im Bereich von 0,01 bis 10 Gew.-%, bezogen auf das Gewicht der Farbmittelzusammensetzung, liegt.

25. Farbmittelzusammensetzung nach einem der Ansprüche 23 oder 24, **dadurch gekennzeichnet, dass** sie in einem Mengenanteil im Bereich von 0,001 bis 20 Gew.-%, bezogen auf die Farbmittelzusammensetzung, und vorzugsweise 0,01 bis 10 % des Gesamtgewichts der Farbmittelzusammensetzung mindestens einen kationischen, anionischen oder nichtionischen ergänzenden Direktfarbstoff enthält, der von dem Farbstoff der Formel (I) verschieden ist.

26. Farbmittelzusammensetzung nach einem der Ansprüche 23 bis 25, **dadurch gekennzeichnet, dass** sie in einem Mengenanteil von 0,0005 bis 12 Gew.-%, bezogen auf das Gewicht der Farbmittelzusammensetzung, und vorteilhaft 0,005 bis 8 Gew.-%, bezogen auf das Gewicht der Farbmittelzusammensetzung, mindestens eine Oxidationsbase enthält.

27. Farbmittelzusammensetzung nach dem vorhergehenden Anspruch, **dadurch gekennzeichnet, dass** sie ferner in einem Mengenanteil von 0,0001 bis 10 Gew.-%, bezogen auf das Gewicht der Farbmittelzusammensetzung, und vorzugsweise 0,005 bis 5 Gew.-%, bezogen auf das Gewicht der Farbmittelzusammensetzung, mindestens einen Kuppler enthält.

28. Farbmittelzusammensetzung nach einem der Ansprüche 23 bis 27, **dadurch gekennzeichnet, dass** sie mindestens ein Oxidationsmittel enthält.

29. Farbmittelzusammensetzung nach dem vorhergehenden Anspruch, **dadurch gekennzeichnet, dass** das Oxidationsmittel unter Wasserstoffperoxid, Alkalimetallperoxiden oder Erdalkalimetallperoxiden; Harnstoffperoxid; Alkalimetallbromaten oder Alkalimetallferricyaniden, Enzymen oder deren Gemischen ausgewählt ist.

30. Farbmittelzusammensetzung nach einem der Ansprüche 28 oder 29, **dadurch gekennzeichnet, dass** der Mengenanteil des Oxidationsmittels im Bereich von 1 bis 20 Gew.-%, bezogen auf das Gewicht der Farbmittelzusammensetzung, und vorzugsweise im Bereich von 1 bis 12 Gew.-%, bezogen auf das Gewicht der Farbmittelzusammensetzung, liegt.

31. Verfahren zum Färben menschlicher Keratinfasern, bei dem die folgenden Schritte durchgeführt werden:
a) die trockenen oder feuchten Fasern werden mit der Zusammensetzung nach einem der Ansprüche 23 bis 29 während einer Zeitspanne in Kontakt gebracht, die für die Bildung der Färbung ausreichend ist,
b) die Fasern werden gegebenenfalls gespült,
c) die Fasern werden gegebenenfalls gewaschen und gespült,
d) die Fasern werden getrocknet oder trocknen gelassen.

32. Vorrichtung mit mehreren Abteilungen für die Durchführung des Verfahrens nach Anspruch 31, wobei eine erste Abteilung eine Farbmittelzusammensetzung aufweist, die mindestens einen Farbstoff der Formel (I) enthält, und eine zweite Abteilung ein Oxidationsmittel enthält.
